# EUROPÄISCHE PATENTANMELDUNG

(11) **EP 3 860 316 A1**
(43) Veröffentlichungstag der Anmeldung: **04.08.2021**
(21) Anmeldenummer: 20154694.2
(22) Anmeldetag: 30.01.2020
(51) Int. Cl.: H05H 1/46, A61B 18/04, A61N 1/44

(54) **PLASMEN, MEDIEN, SPEZIES, SYSTEME, VERFAHREN**

(71) Anmelder: Erbe Elektromedizin GmbH, 72072 Tübingen (DE)
(72) Erfinder: ZENKER, Matthias, 72074 Tuebingen (DE); BRUNECKER, Kristin, 72108 Rottenburg (DE); NEUGEBAUER, Alexander, 72116 Moessingen (DE); WEISS, Martin, 72108 Rottenburg-Seebronn (DE)
(74) Vertreter: Rüger Abel Patentanwälte PartGmbB

(57) **Zusammenfassung**

Erfindungsgemäß wird insbesondere ein Plasma (19) zur Prävention von intraepithelialen Neoplasien, insbesondere durch antivirale Therapie, z.B. gegen das humane Papillomavirus, zur Behandlung von intraepithelialien Neoplasien aller Schweregrade, beispielsweise Carcinoma in situ Läsionen, und/oder zur Behandlung von invasiven epithelial erreichbaren Karzinomen geschaffen, wobei das Plasma erzeugt ist mit einem System (10) zur Erzeugung eines Plasmas (19), bevorzugt eines Argon-Plasmas, mit einem medizinischen Instrument (14) mit wenigstens einer Elektrode (15) mit galvanischem Kontakt zu dem Plasma (19), einem HF-Gerät (11) zur Bereitstellung einer Wechselspannung zur Speisung des Instruments (14) mit elektrischer Leistung und einer Gaszuführungseinrichtung (13), welche eingestellt ist, um das Instrument (14) mit Gas, insbesondere Argon, zu speisen.

## Beschreibung

Die Erfindung betrifft physikalische Plasmen, Medien, Spezies, Systeme und Verfahren zur Behandlung von Krankheiten.

Unter dem Begriff Plasmamedizin sind zahlreiche Verfahren zur Behandlung von Krankheiten mittels physikalischen Plasmas zusammengefasst.

Die Artikel "Cold atmospheric plasma, a novel promising anti-cancer treatment modality" von YAN et al., Oncotarget, 2017, Vol. 8, (No.9), pp: 15977-15995 sowie "Use of cold atmospheric plasma in oncology: a concise systematic review, Dubuc et al., Ther Adv Med Oncol, 2018, Vol. 10: 1-12 beschreiben Forschung zur Anwendung von kalten Plasmen bei Atmosphärendruck (cold atmospheric plasma, CAP) zur Krebsbehandlung.

Der Artikel "Virucide properties of cold atmospheric plasma for future clinical applications", Weiss et al., Journal of Medical Virology 89: 952-959 (2017) beschreibt die Inaktivierung von Viren durch Anwendung von kalten Plasmen bei Atmosphärendruck.

Der Artikel "Physical plasma: a new treatment option in gynecological oncology", Weiss et al., Archives of gynecology and obstetrics, September 2018, skizziert die Verwendung von kaltem physikalischem Plasma bei Atmosphärendruck für die Behandlung und Prävention von gynäkologischen Krebsarten.

Der Artikel "Cold atmospheric plasma (CAP) for anti-cancer applications: Epigenetic effects on DNA integrity and functionality of cervical cancer cells" beschreibt Wirkungen von kaltem Plasma bei Atmosphärendruck auf Krebszellen der Cervix.

Der Artikel "Modifizierte Argon-Plasma-Koagulationsmodi und erste unizentrische klinische Erfahrungen in der gastroenterologischen Endoskopie", Frank et al., Endo heute 2006; 19: 15 - 22 beschreibt die Anwendung von ArgonPlasma-Koagulationsmodi bei der Behandlung von Tumoren und Läsionen.

Der Artikel "Clinical investigation of the safety and efficacy of a cervical intraepithelial neoplasia treatment using a hyperthermia device that uses heat induced by alternating magnetic fields" Koizumi et al., Molecular And Clinical Oncology, 5: 310-316, 2016, beschreibt die Behandlung von Patientinnen mit zervikaler intraepitellialer Neoplasie des Grades III (CIN III) mittels Nadeln, welche zur Behandlung erhitzt wurden.

WO 02/11634 A1 beschreibt einen Hochfrequenzgenerator für die Hochfrequenzchirurgie mit einstellbarer Leistungsbegrenzung. Der Generator erlaubt die Einstellung der Pulsdauer des Modulationssignales und/oder der Pausendauer zwischen den Modulationssignalen so, dass der Spitzenwert der HF-Ausgangsspannung bzw. die Intensität der elektrischen Lichtbögen konstant gehalten wird.

Der Posterbeitrag "Cold atmospheric plasma (CAP) for anti-cancer applications: Epigenetic effects on DNA integrity and functionality of cervical cancer cells", Weiss et al., für den 62. DGGG-Kongress, Berlin, 31.10.-03.11.2018 veranschaulicht den zellwachstumsbehindernden Effekt einer CAP Anwendung auf zervikale SiHa-Zellen.

In dem Artikel "Molecular Effects and Tissue Penetration Depth of Physical Plasma in Human Mucosa Analyzed by Contact- and Marker-Independent Raman Microspectroscopy", Wenzel et al., veröffentlicht am 28. Oktober 2019 in ACS Appl. Mater. Interfaces wird die nicht-invasive Behandlung mit CAP als vielversprechendes therapeutisches Verfahren bei präkanzerösen Läsionen und neoplastischen Erkrankungen der menschlichen Schleimhaut, beispielsweise zervikale Neoplasie, genannt. Der Artikel beschreibt die Ergebnisse einer Studie mit dem Ziel die Verwendung von Raman-Mikrospektroskopie zu untersuchen, um Plasmaeffekte auf menschliches Gewebe zu charakterisieren, was an Zervixgewebeproben ex-vivo untersucht wurde. Wie beschrieben wird in einem Teil der Studie die Wirkung von CAP, Zellvermehrung zu behindern, an einer Zellkultur mit einer Zelllinie von Krebszellen der Zervix bestätigt. Die CAP-Behandlung sowohl der Zervixgewebeproben als auch der Zelllinie wurde mit einem nach dem Jet-Prinzip arbeitenden Instrument zur Argonplasmaerzeugung durchgeführt. Im Rahmen der Studie wurde das Instrument dynamisch über die Proben bewegt, um thermische Gewebeschädigung auszuschließen.

Die Website https://clinicaltrials.gov/ct2/show/NCT03218436?term=argon+plasma&cond=CIN&ran k=1 beschreibt eine Studie zur Behandlung von zervikaler intraepithelialer Neoplasie mit physikalischem Plasma niedriger Temperatur. Als Einschlusskriterium für die Plasmabehandlung wird histologisch gesicherte CIN Grad I / II genannt.

Vor diesem Hintergrund besteht ein Bedarf an verbesserten Plasmen zur Behandlung von Krankheiten sowie Systemen zur Erzeugung solcher Plasmen und Verfahren.

Zur Lösung dieser Aufgabe schafft die Erfindung insbesondere ein Plasma gemäß Anspruch 1, ein Medium nach Anspruch 8, Spezies nach Anspruch 9, Systeme nach Anspruch 10 bzw. 11 sowie ein Verfahren nach Anspruch 14.

Erfindungsgemäß wird gemäß einem ersten Aspekt der Erfindung Plasma zur Prävention von intraepitelialen Neoplasien, insbesondere durch antivirale Therapie, z.B. durch plasmabasierte Inaktivierung des humanen Papilloma-Virus, zur Behandlung von intraepitelialen Neoplasien aller Schweregrade, beispielsweise Carcinoma in situ Läsionen und/oder zur Behandlung von invasiven epithelial erreichbaren Karzinomen geschaffen. Das Plasma ist bevorzugt ein Argonplasma, andernfalls beispielsweise ein Heliumplasma. Das System zur Erzeugung des Plasmas weist ein medizinisches Instrument mit wenigstens einer Elektrode, ein HF-Gerät zur Bereitstellung einer Wechselspannung zur Speisung des Instruments mit elektrischer Leistung und eine Gaszuführungseinrichtung auf, welche eingestellt ist, um das Instrument mit Gas, insbesondere Argon, zu speisen. Wenigstens eine Elektrode des Systems hat vorzugsweise galvanischen Kontakt zu dem Plasma. Das System erzeugt das Plasma bevorzugt als ein stromführendes Plasma.

Ein zweiter Aspekt der Erfindung betrifft mittels des Plasmas aktivierte Medien. Aktiviert bedeutet, dass mittels des Plasmas in dem Medium therapeutisch wirksame Spezies, insbesondere Radikale, erzeugt und/oder in das Medium therapeutisch wirksame Spezies, insbesondere Radikale, eingebracht sind. Bei den Medien kann es sich beispielsweise um Flüssigkeit, insbesondere Suspension, Emulsion, insbesondere Gewebeflüssigkeit, menschliches oder tierisches Gewebe, insbesondere neoplastisches Gewebe, oder beispielsweise pastöses oder festes Material handeln. Die Medien, insbesondere Materialien, können zur Behandlung von intraepithelialen Neoplasien herangezogen werden. Ein aktiviertes Medium, welches zu behandelndes körpereigenes Gewebe des Patienten aufweist, oder ein aktiviertes körpereigenes Medium an dem zu behandelnden körpereigenen Gewebe, z.B. Körperflüssigkeit, kann auch als aktiviertes Zielmedium bezeichnet werden. Ein aktiviertes körperfremdes Medium, z.B. eine Flüssigkeit, insbesondere eine Lösung, Suspension oder Emulsion, ein Gel oder eine Paste, welches zur Behandlung auf oder in Gewebe des Patienten appliziert wird oder welches dem Patienten auf andere Art zugeführt wird, kann als Medikament zur Prävention oder Behandlung der genannten Krankheiten aufgefasst werden. Wenn das Plasma gemäß dem ersten Aspekt zur Herstellung eines aktivierten körpereigenen oder körperfremden Mediums verwendet wird, kann das Plasma in diesem Sinne mittelbar zur Behandlung insbesondere einer oder mehrerer der im Zusammenhang mit dem ersten Aspekt genannten Krankheiten eingesetzt werden. Bei den Radikalen kann es sich beispielsweise um reaktive Sauerstoffspezies (reactive oxygen species, ROS) und/oder reaktive Stickstoffspezies (reactive nitrogen species, RNS), handeln. Die Gesamtheit aller reaktiven Sauerstoffspezies und aller reaktiven Stickstoffspezies wird als RONS bezeichnet.

Ein dritter Aspekt der Erfindung betrifft mittels des Plasmas erzeugte Spezies, insbesondere in oben genanntes Medium eingebrachte Spezies und/oder in oben genanntem Medium erzeugte Spezies, insbesondere Radikale.

In einem vierten Aspekt der Erfindung wird ein System zur Erzeugung des Plasmas, bevorzugt stromführenden Plasmas, gemäß dem ersten Aspekt, bevorzugt eines Argonplasmas, angegeben. Das System weist ein medizinisches Instrument mit wenigstens einer Elektrode mit vorzugsweise galvanischem Kontakt zu dem Plasma und ein HF-Gerät zur Bereitstellung einer Wechselspannung zur Speisung des Instruments mit elektrischer Leistung auf. Zudem weist das System eine Gaszuführungseinrichtung auf, welche zur Herstellung des Plasmas einstellbar ist, das Instrument mit Gas, insbesondere Argon, zu speisen. Das System kann in Ausführungsformen durch Wahl bestimmter Systemeinstellungen, wie z.B. HF-Spannung, HF-Leistung, Modulation einer HF-Frequenz, Gasfluss und dergleichen, zur Erzeugung des Plasmas und damit zur Vorbeugung und/oder Behandlung der genannten Krankheiten bestimmt sein.

Wenigstens eine Elektrode des Systems kann galvanischen Kontakt mit dem Plasma haben, indem eine elektrisch leitfähige, insbesondere metallische, Oberfläche der Elektrode Kontakt zu dem Plasma aufweist. Unter einem stromführenden Plasma wird ein Plasma mit Elektrodenkontakt verstanden, so dass Elektronen von der Elektrode in das Plasma übertreten. Anders als bei einer Barriereentladung durch eine dielektrische Schicht auf der Elektrode, so dass das Plasma keinen Elektrodenkontakt aufweist, treten bei bevorzugten Ausführungsformen des erfindungsgemäßen Systems zur Behandlung der genannten Krankheiten, z.B. durch Vorbeugung, Elektronen aus der Elektrode in das Plasma ein. Mit den bevorzugten Ausführungsformen des erfindungsgemäßen Systems lässt sich die Entladung nahe an die zu behandelnde Stelle heranführen, was zu einer hohen Dichte an neutralen und geladenen, auch kurzlebigen Spezies, beispielsweise reaktiven Sauerstoffspezies und/oder reaktiven Stickstoffspezies, wozu insbesondere Radikale gehören können, in dem Plasma an der Gewebestelle und/oder in der zu behandelnden Gewebsstelle führt.

Mit dem System lässt sich in Ausführungsformen ein warmes Plasma erzeugen, das die nicht-thermische Applikation des Plasmas durch Bewegen über die zu behandelnde Stelle erlaubt, beim Verharren an einer Stelle aber zu einem thermischen Effekt, insbesondere zu einer Beschädigung der funktionellen Gewebestruktur, insbesondere durch Denaturierung von Proteinen, führen kann. Das warme Plasma kann beispielsweise eine Temperatur, insbesondere Ionentemperatur, von größer 45°C, größer 55°C oder sogar größer 65°C aufweisen. Insbesondere kann an der Stelle, benachbart zu welcher die Spitze des Instruments gehalten wird, ohne diese über das Gewebe zu bewegen, die Temperatur an der Oberfläche der Gewebestelle auf wenigstens 45° Celsius, wenigstens 55° Celsius oder sogar wenigstens 65° Celsius steigen. Das Plasma kann beispielsweise eine Temperatur aufweisen, so dass erst bei einer Applikationsgeschwindigkeit einschließlich oder oberhalb eines Grenzwerts, beispielsweise 10 mm/s, mit welcher das Gewebe überstrichen wird, die Gewebetemperatur an der zu behandelnden Gewebestelle des menschlichen oder tierischen Patienten kleiner oder gleich einer Grenztemperatur, beispielsweise 40°C, besonders bevorzugt kleiner oder gleich 37°C bleibt. Um bevorzugt mit einer händisch erreichbaren und kontrollierbaren Applikationsgeschwindigkeit von bevorzugt maximal 50 mm/s oder kleiner arbeiten zu können, so dass die Gewebetemperatur an der Gewebestelle, welche mit dem Plasma überstrichen wird, kleiner oder gleich einer Grenztemperatur, beispielsweise 40°C, besonders bevorzugt kleiner oder gleich 37°C bleibt bzw. so dass eine thermische Gewebeschädigung, insbesondere Koagulation, durch die Plasmabehandlung ausbleibt, weist das Plasma bevorzugt eine Temperatur von kleiner oder gleich einer Grenztemperatur auf, wobei die Grenztemperatur beispielsweise 150°C oder 120°C sein kann.

Gemäß einem fünften Aspekt der Erfindung ist ein Verfahren geschaffen, wobei das Verfahren die Verwendung eines Plasmas, beispielsweise des Plasmas gemäß dem ersten Aspekt, zur Prävention von intraepitelialen Neoplasien, insbesondere durch antivirale Therapie, z.B. gegen das humane Papilloma-Virus, zur Behandlung von intraepitelialen Neoplasien aller Schweregrade, beispielsweise Carcinoma in situ Läsionen, oder zur Behandlung von invasiven epithelial erreichbaren Karzinomen umfasst. Gleichbedeutend mit der Verwendung des Plasmas gemäß dem ersten Aspekt, und damit auch zur Erfindung gehörend, ist die Verwendung des Mediums (z.B. die Erzeugung des Mediums), der Spezies bzw. des Systems gemäß der Aspekte zwei bis vier für ein oder mehrere der genannten Behandlungen.

Das Verfahren umfasst insbesondere die nicht-thermische Verwendung eines warmen Plasmas, beispielsweise Ausführungsformen des Plasmas gemäß dem ersten Aspekt. Dazu wird ein Instrument, beispielsweise das Instrument des Systems gemäß dem vierten Aspekt, wie vorstehend beispielhaft erläutert, und damit das Plasma bevorzugt derart zügig über das zu behandelnde Gewebe geführt, ohne es dabei zu berühren, dass die Temperaturen der derart mit dem Plasma überstrichenen Gewebestellen unter einer Grenztemperatur, bevorzugt 37°C oder 40°C, bleiben, so dass eine thermische Schädigung des Gewebes sicher ausgeschlossen ist.

Aufgrund der ausbleibenden Gewebedestruktion bei der nicht-thermischen Anwendung des Plasmas werden klassische Risiken und Komplikationen von Laserbehandlung (Blutung, Infektion) und Konisation (Blutung, Infektionen, CK-Verkürzung, 10-faches Risiko für Schwangerschafts-, Geburtskomplikationen usw.) vermieden. Die nicht-thermische Behandlung ist schmerzfrei bzw. schmerzarm und kann deshalb ambulant sowie ohne allgemeine Sedierung und ohne Lokalanästhesie in der üblichen Sprechstundensituation durchgeführt werden. In vielen Fällen kann bereits eine Behandlung mit einer Behandlungszeit von 10 Sekunden bis 10 Minuten ausreichend sein. Für die Behandlung ist nur eine Person (medizinisches Fachpersonal oder Arzt) erforderlich. Sport, Geschlechtsverkehr, Baden, Schwimmen, Erwerbstätigkeit sind unmittelbar nach der Behandlung wieder möglich.

Gemäß einem sechsten Aspekt der Erfindung wird ein System zur Erzeugung eines Plasmas, bevorzugt eines stromführenden Plasmas, bevorzugt eines Argon-Plasmas, mit einem medizinischen Instrument mit wenigstens einer Elektrode und einem HF-Gerät zur Bereitstellung einer Wechselspannung zur Speisung des Instruments mit elektrischer Leistung angegeben. Die Elektrode hat bevorzugt galvanischen Kontakt zu dem Plasma. Das System weist bevorzugt eine Gaszuführungseinrichtung auf, welche dazu eingerichtet ist, um das Instrument zur Erzeugung eines Plasmas mit Gas, insbesondere Argon, zu speisen. Das System weist eine Vorrichtung zur Regelung der Ausgangsleistung, z.B. der Ausgangswirkleistung oder der Ausgangsscheinleistung oder des Mittelwerts der Ausgangsmomentanleistung, des HF-Geräts auf einen Sollwert auf. Das System ist bevorzugt dazu bestimmt, dies kann in Ausführungsformen durch Wahl bestimmter Systemeinstellungen wie z.B. Spannung, Leistung, Modulation einer HF-Frequenz, Gasfluss und dergleichen, erfolgt sein, zu wenigstens einer der folgenden Therapien eingesetzt zu werden: zur Prävention von intraepitelialen Neoplasien, insbesondere durch antivirale Therapie, z.B. gegen das humane Papilloma-Virus, zur Behandlung von intraepitelialen Neoplasien aller Schweregrade, beispielsweise Carcinoma in situ Läsionen und/oder zur Behandlung von invasiven epithelial erreichbaren Karzinomen.

Gemäß einem siebten Aspekt der Erfindung wird ein Plasma zur Verwendung zur Prävention von intraepitelialen Neoplasien, insbesondere durch antivirale Therapie, z.B. gegen das humane Papilloma-Virus, Behandlung von intraepitelialen Neoplasien aller Schweregrade, beispielsweise Carcinoma in situ Läsionen und/oder zur Behandlung von invasiven epithelial erreichbaren Karzinomen erzeugt mittels des Systems gemäß dem sechsten Aspekt angegeben. Ein achter Aspekt der Erfindung betrifft ein Medium aktiviert mit einem Plasma gemäß dem siebten Aspekt zu den angegebenen therapeutischen Behandlungen. Ein neunter Aspekt der Erfindung betrifft Spezies erzeugt mittels eines Plasmas gemäß dem siebten Aspekt. Ein zehnter Aspekt betrifft ein Verfahren zur Prävention von intraepitelialen Neoplasien, insbesondere durch antivirale Therapie, z.B. gegen das humane Papilloma-Virus, Behandlung von intraepitelialen Neoplasien aller Schweregrade, beispielsweise Carcinoma in situ Läsionen und/oder zur Behandlung von invasiven epithelial erreichbaren Karzinomen mit einem System gemäß dem sechsten Aspekt, einem Plasma gemäß dem siebten Aspekt, einem Medium gemäß dem achten Aspekt und/oder mit Spezies gemäß dem neunten Aspekt. Ausführungsformen des Verfahrens gemäß dem zehnten Aspekt betreffen insbesondere die nicht-thermische Verwendung eines Plasmas gemäß dem siebten Aspekt. Damit in Ausführungsformen gleichbedeutend und zur Erfindung gehörend ist die Verwendung des Systems gemäß dem sechsten Aspekt, der Medien gemäß dem achten Aspekt sowie der Spezies gemäß dem neunten Aspekt.

Hierin angegebene Merkmale des Plasmas, Systems, Mediums, der Spezies bzw. des Verfahrens gemäß den ersten bis fünften Aspekten der Erfindung können mit ihren Vorteilen optionale Merkmale des Plasmas, Systems, Mediums, der Spezies bzw. des Verfahrens gemäß den sechsen bis zehnten Aspekten der Erfindung sein und umgekehrt. Ausführungsformen des Systems gemäß dem sechsten Aspekt, des Plasmas gemäß dem siebten Aspekt, des Mediums gemäß dem achten Aspekt und der Spezies gemäß dem neunten Aspekt entsprechen dem Plasma, System, Medium bzw. Spezies gemäß den ersten bis vierten Aspekten der Erfindung, so dass die Beschreibung zu den ersten bis fünften Aspekten zur Beschreibung insbesondere dieser Ausführungsformen heranzuziehen ist.

Weitere vorteilhafte Ausführungsformen und Merkmale der erfindungsgemäßen Plasmen, Medien, Spezies, Systeme und Verfahren gemäß dem ersten bis zehnten Aspekt ergeben sich aus nachfolgender Beschreibung. Auch wenn im Folgenden Plasma, Medium, System, Verfahren in der Einzahl genannt ist, sind stets optionale Merkmale und Vorteile aller Aspekte, des ersten Aspekts bis zehnten Aspekts, der Erfindung angesprochen, es sei denn, aus dem Zusammenhang ergibt sich etwas anderes:

Mit dem erfindungsgemäßen Plasma lassen sich insbesondere intraepiteliale Neoplasien wenigstens eines der folgenden Organsysteme von Säugern behandeln: Cervix uteri, Os, Ösophagus, Gaster, Colon, Rektum und Peritoneum.

Das Plasma kann insbesondere zur Behandlung von intraepitelialen Neoplasien, insbesondere von zervikalen intraepithelialen Neoplasien, des Grades I oder II, beim Menschen verwendet werden.

Trotz sinkender Inzidenzen hierzulande aufgrund größter Anstrengungen in Prävention und frühzeitiger radikaler Therapie erkranken jährlich weltweit über 500.000 Frauen an invasivem Cervixkarzinom (CC). Pro Jahr sterben etwa 270.000 Patientinnen an der Erkrankung und ein Großteil aller Patientinnen kämpft ein Leben lang mit den mitunter schweren körperlichen und seelischen Folgen der Erkrankung und Therapie.

Zervikale Carcinoma in Situ (CIS) und CC entstehen aus Vorläuferläsionen, die über einen Zeitraum von meist mehreren Jahren persistieren. Neben anderen ist der Hauptrisikofaktor für die Entstehung dieser zervikalen intraepitelialen Neoplasien (CIN) die lokale Infektion mit high-risk Varianten des humanen Papilloma-Virusses (HPV).

Die primäre Therapie der CIN besteht aus Laserevaporiation (thermische Verödung) oder Konisation (Gewebeentfernung). Diese Verfahren werden zumeist in Vollnarkose durchgeführt und sind mit relativ hoher Invasivität sowie großem klinischem und finanziellem Aufwand verbunden. Die hohe Inzidenz der CIN von jährlich etwa 4,2 Neuerkrankungen pro Tausend Individuen ergibt eine große Menge an teuren OP-Leistungen. Zusätzlich sind diese operativen Eingriffe zur Behandlung von CIN unter Umständen mit schweren Blutungen, verminderter Fertilität und einem vielfach erhöhten Risiko für Schwangerschaftskomplikationen assoziiert. Der Spagat zwischen Übertherapie und Sorge vor der Manifestation eines invasiven CC ist ein gesundheitsökonomisches Problem. CIN werden aufgrund unzureichender HPV-Impfquoten und anderer Risikofaktoren in Deutschland und weltweit auch weiterhin ein ernstzunehmendes Problem darstellen. Wirksame und gering-invasive Verfahren ohne Narkotisierung zur Behandlung, insbesondere der CIN, aber auch für andere Schleimhautpräkanzerosen sind im klinischen Alltag dringend erforderlich und derzeit größtenteils vakant.

Die Erfindung schafft hier Abhilfe, indem diese Plasmen, Systeme, Medien, Spezies und Verfahren zur besonders schonenden aber wirksamen Therapie von insbesondere CIN schafft. Bevorzugt werden die Plasmen, Systeme, Medien, Spezies zur nicht-thermischen Behandlung von Gewebeoberflächen bis in das Gewebe hinein verwendet. Dies bedeutet, dass die Temperatur an der behandelten Gewebestelle besonders bevorzugt stets unter einer kritischen Temperatur für thermische Gewebeschädigung bleibt, beispielsweise kleiner oder gleich 40°C oder kleiner oder gleich 37°C.

Der Gasfluss des Gases (Plasmagas), z.B. Argon, dient dazu eine geeignete, möglichst definierte, Mischatmosphäre zwischen distaler Instrumentenspitze und Gewebe zu schaffen, so dass eine Zündung des Plasmas möglich ist. Bei zu niedrigem Gasfluss befindet sich zu wenig Plasmagas zwischen Elektrode und Gewebe. Der Mindestgasfluss ist derjenige Gasfluss, der mindestens durch das Instrument strömen muss, damit ein Plasma zündet. Bei zu hohem Gasfluss wird durch Turbulenz zu viel Luft oder anderes Umgebungsgas oder -medium zugemischt. Der Höchstgasfluss ist derjenige Gasfluss, der höchstens durch das Instrument strömen darf, damit ein Plasma zündet. Eine reine Plasmagas-Atmosphäre kann mutmaßlich die Bildung von therapeutisch wirksamen Spezies erschweren. Daher liegt der gewählte Gasfluss des Gases (Plasmagas), z.B. Argon, zur Erzeugung des Plasmas vorzugsweise in einem Bereich bei dem Mindestgasfluss oder in einem Bereich bei dem Höchstgasfluss. Der gewählte Gasfluss, der durch das Instrument strömt, kann beispielsweise wenigstens so groß sein wie der Mindestgasfluss, aber kleiner als der Höchstgasfluss. Der Gasfluss an Plasmagas wird vorzugsweise in einem Bereich von einschließlich dem Mindestgasfluss bis einschließlich dem Gasfluss gewählt, der z.B. drei Mal größer ist als der Mindestgasfluss. Der Mindestgasfluss, und auch der Höchstgasfluss, können von mehreren Parametern und/oder Einstellungen abhängen. Der Mindestgasfluss kann insbesondere von einem gewünschten Behandlungsabstand zwischen Instrumentenspitze und Gewebeoberfläche abhängen. Für die Behandlung kann beispielsweise der Behandlungsabstand zwischen Instrumentenspitze und Gewebeoberfläche (z.B. 7 Millimeter) festgelegt und an Hand des festgelegten Behandlungsabstandes der Gasfluss gewählt werden, der durch das Instrument strömen soll. Bei einem Innendurchmesser des Gaskanals am distalen Ende von 2,4 mm beispielsweise kann der Gasfluss bevorzugt in einem Bereich von einschließlich 1 l/min und einschließlich 3 l/min, besonders bevorzugt von einschließlich 1,3 l/min bis einschließlich 2,5 l/m, beispielsweise 1,6 l/min ± 20% sein. Die Angaben beziehen sich - wie die weiteren Angaben zu Gasflüssen in dieser Anmeldung auch - auf Normalbedingungen. Normalbedingungen liegen bei einer Temperatur von 0°C und einem Atmosphärendruck von 1 atm vor. Bei kleinerem Innendurchmesser kann, bei ansonsten gleichen Parametern und Einstellungen, ein kleinerer Gasfluss, bei größerem Innendurchmesser, bei ansonsten gleichen Parametern und Einstellungen, ein größerer Gasfluss vorteilhaft sein. Der Innendurchmesser der Gaszuführung (z.B. ein Gaskanal) des Instruments an dessen distalem Ende kann beispielsweise von einschließlich 0,5 mm bis einschließlich 10 mm, bevorzugt von einschließlich 0,8 mm bis einschließlich 3 mm, insbesondere 2,4 mm betragen.

In Ausführungsformen kann ein Gasfluss von beispielsweise Argon oder einem anderen Plasmagas von bevorzugt kleiner 3 l/min, besonders bevorzugt kleiner oder gleich 2 l/min, vorteilhaft sein und einerseits zu einem sicheren Zünden des Plasmas auch bei geringer Spannung und andererseits zu einem Gemisch an Argon und Luft zwischen der Elektrode und dem Gewebe führen, so dass vermittelt über das Plasma eine hohe Dichte an reaktiven Spezies, insbesondere reaktiven Sauerstoffspezies und/oder reaktiven Stickstoffspezies entstehen, die mit dem Gasstrom zu dem Gewebe hingetragen und/oder in das Gewebe eingetragen werden.

Zur Herstellung eines wirksamen Plasmas werden Instrumente bevorzugt, bei denen die elektrische Stromflussrichtung mit der Plasmaströmungsrichtung zum Gewebe übereinstimmt.

Ein besonders wirksames Plasma lässt sich mittels eines Systems erzeugen, wenn dieses eine Neutralelektrode aufweist, wobei die Neutralelektrode an dem Körper eines Patienten angeordnet ist, um den Stromkreis von dem HF-Gerät über die Elektrode durch das Plasma und den Körper des Patienten zu schließen. Das Plasma ist zwischen der Elektrode am distalen Ende des Instruments und dem Gewebe des Patienten gezündet. Wenn das System zur Herstellung eines aktivierten Mediums außerhalb oder gesondert von dem Körper des Patienten mittels des Plasmas verwendet werden soll, kann das Medium auf einem Träger oder in einem Behälter angeordnet werden, der als Neutralelektrode dient. Z.B. kann eine Neutralelektrodeneinheit des Systems mit einem elektrisch leitfähigen Träger oder Behälter für das Medium elektrisch leitfähig verbunden sein.

Eine besonders schonende Behandlung ist möglich, wenn das HF-Gerät eingestellt ist, eine auf maximal 3,5 Watt, besonders bevorzugt maximal 2,5 Watt, ganz besonders bevorzugt maximal 2 Watt, noch weiter bevorzugt maximal 1 Watt oder kleiner begrenzte elektrische Ausgangswirkleistung abzugeben. Mit einer Begrenzung der Ausgangswirkleistung auf einen maximalen Wert kann die tatsächliche Leistung den maximalen Wert, z.B. 2 Watt, oder einen geringeren Wert annehmen, beispielsweise 1 Watt.

In bevorzugten Ausführungsformen ist das HF-Gerät eingestellt, die Elektroden mit einer Wechselspannung zu speisen, die eine Hochfrequenz (HF) von mindestens 100 kHz, vorzugsweise zwischen einschließlich 200 kHz bis einschließlich 16 MHz, besonders bevorzugt zwischen 300 kHz und 500 kHz, beispielsweise 350 kHz, aufweist. Die HF-Frequenz kann auch als Trägerfrequenz bezeichnet werden.

Die Wechselspannung kann mit einer festen oder variablen Mittelfrequenz gepulst sein (Modulation mit einer Mittelfrequenz), wobei die Mittelfrequenz beispielsweise zwischen einschließlich 5 kHz und einschließlich 100 kHz, besonders bevorzugt zwischen einschließlich 10 kHz und einschließlich 50 kHz, beispielsweise 20 kHz, beträgt. Die Pulslänge jedes der Mittelfrequenzpulse beträgt vorzugsweise eine oder mehrere HF-Perioden. Mit der Pulsung mit der Mittelfrequenz kann die Leistung des HF-Geräts gegenüber einem Dauerstrichbetrieb (continuous wave) vermindert werden, ohne die Spitzenspannung unter ein für die Zündung kritisches Maß senken zu müssen.

Die Wechselspannung ist außerdem bevorzugt mit einer festen oder variablen Niederfrequenz gepulst (Modulation mit einer Niederfrequenz), welche beispielsweise zwischen einschließlich 0,5 Hz und einschließlich 200 Hz, vorzugsweise zwischen einschließlich 20 Hz und einschließlich 150 Hz, insbesondere 100 Hz, beträgt. Die Pulslänge jedes der Niederfrequenzpulse beträgt vorzugsweise mindestens eine Mittelfrequenzperiode, vorzugsweise zwischen einschließlich einer und einschließlich 50 Mittelfrequenzperioden, beispielsweise 20 Mittelfrequenzperioden. Enthält die Pulslänge eines Niederfrequenzpulses mehr als einen Mittelfrequenzpuls, kann auch von einem Niederfrequenzpulspaket gesprochen werden. Die Pulsung mit der Niederfrequenz führt zusätzlich oder alternativ zu der Mittelfrequenzpulsung zu einer Verminderung der Ausgangswirkleistung gegenüber einem Dauerstrichbetrieb des HF-Geräts. Das HF-Gerät kann einen Dauerstrichbetrieb zulassen oder nicht zulassen. Zusätzlich führt die Pulsung mit der Niederfrequenz aber zu einem Erlöschen des Plasmas in den Pulspausen zwischen den Niederfrequenzpulsen und folglich zu einem Ablösen des Plasmas von der Gewebeoberfläche. Das Plasma kann damit über die Gewebeoberfläche geführt werden, ohne an der Gewebeoberfläche hängen zu bleiben. Damit wird die Erzielung eines besonders gleichmäßigen Behandlungsergebnisses erleichtert. Auch wird damit die Vermeidung von heißen Gewebestellen, d.h. Gewebestellen mit einer behandlungsbedingten Temperatur oberhalb einer kritischen Temperatur von beispielsweise 37 °C oder 40°C, durch "Anhaftung" des Plasmas an einer Stelle bei gleichzeitig intensiver Behandlung der Gewebestellen erleichtert.

Das System kann dazu eingerichtet sein, zu Beginn eines Niederfrequenzpulses, welcher insbesondere ein Paket aus mehreren Pulsen der Mittelfrequenz sein kann, die Spitzenausgangsspannung des HF-Geräts so lange zu erhöhen, bis entweder eine Obergrenze erreicht ist oder ein Plasma gezündet wird. Die Obergrenze beträgt beispielsweise zwischen einschließlich 2 kV und 6 kV, bevorzugt zwischen 4 kV und 5 kV, beispielsweise 4,7 kV. Das System ist vorzugsweise weiter dazu eingerichtet, dass bei von dem System erkannter Zündung die Spannung des Leistungsnetzteils des HF-Geräts abgesenkt wird. Dadurch wird eine Verminderung der Spitzenausgangsspannung des HF-Geräts erreicht, so dass zu Beginn des folgenden Niederfrequenzpulses die Spitzenspannung geringer ist als zum Zeitpunkt der Plasmazündung im Niederfrequenzpulspaket zuvor. Dadurch kann die Erzeugung eines zu intensiven Plasmas vermieden werden, das zu einem zu starken thermischen Effekt, insbesondere zu einer thermischen Gewebeschädigung führen könnte.

Das System weist vorzugsweise eine Vorrichtung zur Regelung der Ausgangsleistung, beispielsweise der Ausgangswirkleistung oder der Ausgangsscheinleistung, des HF-Geräts auf einen Sollwert auf, wobei das erfindungsgemäße Plasma bevorzugt bei aktivierter Regelung der Ausgangsleistung erzeugt ist.

Die Vorrichtung zur Regelung der Ausgangsleistung kann beispielsweise dazu eingerichtet sein, die Spannungsamplitude derart anzupassen, dass ein Sollwert einer Ausgangsleistung des HF-Geräts erreicht wird.

Bei einer Modulation mit einer Mittelfrequenz und einer Niederfrequenz weist ein Pulspaket der Niederfrequenz bevorzugt einen oder mehrere Pulse der Mittelfrequenz auf, welches gefolgt wird von einer Pulspause. Ein Pulspaket der Mittelfrequenz weist bevorzugt eine oder mehrere Schwingungsperioden der Hochfrequenz auf, gefolgt von einer Pause. Insbesondere bei kleinen und mittleren Ausgangsleistungen sind die Pulspakete der Niederfrequenz so kurz, dass eine Regelung der Leistung über bekannte Regelstrategien (Spannungsregelung) nicht möglich ist. Ohne eine Gegenmaßnahme ist dann die in das Gewebe eingebrachte Leistung von der Spannung abhängig, bei der das Plasma zündet. Diese Zündspannung hängt von den äußeren Bedingungen, z.B. Abstand zum Gewebe, Beschaffenheit der Elektrode oder verwendetes Plasmagas (z.B. Helium oder Argon) ab. Die Auswirkung dieses Zusammenhangs führen für viele Anwendungen nicht notwendig zu einem klinisch relevanten Unterschied im Gewebeeffekt. Jedoch wäre ein vom Abstand unabhängiger Gewebeeffekt insbesondere bei der Behandlung von CIN-Läsionen von besonderem Vorteil. Vorzugsweise wird daher alternativ oder zusätzlich zu der Anpassung der Spitzenspannung die Pulsung (Modulation) der Ausgangsspannung angepasst. Die Ausgangleistung kann beispielsweise durch Verändern der Modulation der Mittelfrequenz und/oder der Niederfrequenz und/oder durch Veränderung der Länge der Niederfrequenzpulspakete beeinflusst werden, um die Ausgangsleistung zu regeln.

Die Vorrichtung kann folglich dazu eingerichtet sein, bei der Erzeugung des Plasmas die Ausgangsleistung durch Anpassung entweder der Spitzenspannung oder der Anpassung der Modulation oder durch beide Maßnahmen zu regeln.

Die Modulationsveränderung, wie sie die Erfindung bevorzugt anstelle der Anpassung der Spitzenspannung zur Anpassung der Ausgangsleistung vorsieht, kann die Niederfrequenz und/oder die Mittelfrequenz betreffen. Die Ausgangsleistung kann kontinuierlich oder in regelmäßigen oder unregelmäßigen Abständen erfasst werden. Sie wird vorteilhaft nach Ende eines Pulspakets der Niederfrequenz erfasst und mit dem Sollwert verglichen. Je nach Abweichung vom Sollwert können die Pulslänge und/oder Periode der Mittelfrequenz und/oder der Niederfrequenz so verändert werden, dass sich die Ausgangsleistung dem Sollwert annähert.

Der Sollwert der gemittelten Ausgangswirkleistung kann beispielsweise maximal 3,5 W, besonders bevorzugt maximal 2,5 W, ganz besonders bevorzugt maximal 2 W, noch weiter bevorzugt 1 W oder weniger betragen. Wenn der Sollwert der gemittelten Ausgangswirkleistung beispielsweise maximal 2 W beträgt, kann der tatsächlich gewählte Sollwert beispielsweise 2 W oder kleiner, z.B. 1 Watt, betragen. Die Leistungsangabe kann sich beispielsweise auf eine Mittelung über eine Periode der Niederfrequenz beziehen. Alternativ kann sich die Leistungsangabe beispielsweise auf eine Mittelung über die Pulsdauer der Niederfrequenz beziehen. Oder die Leistungsangabe kann sich beispielsweise auf eine Mittelung über wenigstens einen oder mehrere Mittelfrequenzpulse oder -perioden beziehen. Der Sollwert ist bevorzugt kleiner als die maximale HF-Ausgangswirkleistung, die von dem HF-Gerät abgegeben werden kann.

Weitere Vorteile und Merkmale des erfindungsgemäßen Plasmas, der erfindungsgemäßen Systeme, Medien, Spezies und Verfahren ergeben sich aus nachfolgender Beschreibung sowie den Figuren. Auch wenn im Folgenden Plasma, Medium, System, Verfahren in der Einzahl genannt ist, sind stets optionale Merkmale und Vorteile aller Aspekte, des ersten Aspekts bis zehnten Aspekts, der Erfindung angesprochen, es sei denn, aus dem Zusammenhang ergibt sich etwas anderes.

Es zeigen:
Figur 1a - eine schematische Ansicht eines Systems mit einem monopolaren Instrument und einer Neutralelektrode,
Figur 1b - ein alternatives System mit einem Instrument zur Erzeugung eines erfindungsgemäßen Plasmas nach dem Jet-Prinzip,
Figur 2 - durch Behandlung mittels eines Systems mit monopolarem Instrument mit Neutralelektrode bzw. mittels eines Systems mit einem nach dem Jet-Prinzip arbeitenden Instruments erreichte Spindichten,
Figuren 3, 4 -schematische Darstellungen der Verwendung eines Plasmas erzeugt mittels eines erfindungsgemäßen Systems,
Figur 5 - eine Gewebeoberflächentemperatur einer Hydrogelprobe während einer Applikation von atmosphärischem Argonplasma mit unterschiedlichen Applikationsgeschwindigkeiten,
Figur 6 - durchschnittliche absolute Zellzahlen nach 24, 72 und 120 Stunden bei nicht-thermischer Applikation von atmosphärischem Argonplasma auf Cervixkarzinomzelllinien In-vitro mit unterschiedlichen Dosierungen von 0 Sekunden bis 120 Sekunden,
Figur 7 - eine schematische Darstellung zur Veranschaulichung von HF-Generatoren erfindungsgemäßer Systeme, z.B. für ein System gemäß Figur 1a oder Figur 1b, bevorzugt mit einer Vorrichtung zur Regelung der Ausgangsleistung des HF-Generators,
Figur 8a, Figur 8b -schematische Darstellungen einer Pulsabfolge der HF-Ausgangsspannung des HF-Generators gemäß Figur 7.

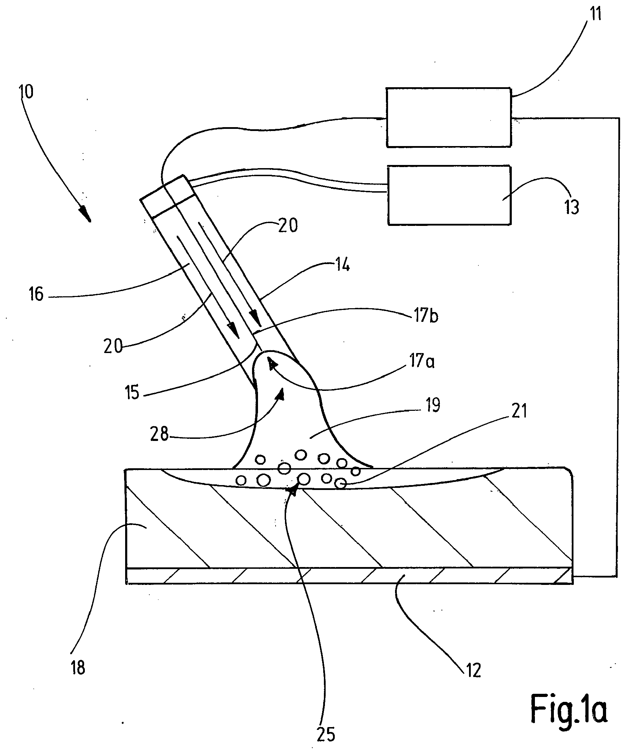

In Figur 1a ist ein Beispiel einer Ausführungsform eines erfindungsgemäßen Systems 10 dargestellt. Das System 10 weist ein HF-Gerät 11, eine Neutralelektrode 12, eine Gaszuführungseinrichtung 13 und ein medizinisches Instrument 14 mit einer Elektrode 15, insbesondere Metallelektrode, auf. Die Elektrode 15 kann beispielsweise aus Edelstahl, Wolfram oder einem anderen elektrisch leitfähigen Material bestehen. Ausführungsbeispiele sind möglich, in denen die Elektrode 15 zumindest eine metallische Oberfläche auf einem nicht-metallischen Kern aufweist. Die Elektrode 15 kann insbesondere die Form eines Drahtes, eines Plättchens oder eines Spatels haben. Das distale Ende 17a der Elektrode 15 kann beispielsweise abgerundet, zylinderförmig oder mit einer Spitze versehen ausgebildet sein. Die Elektrode kann insbesondere ein Edelstahlplättchen mit einer Spitze am distalen Ende sein. Die Elektrode 15 des Instruments 14 ist vorzugsweise frei von einer dielektrischen Beschichtung. Jedenfalls aber ist das distale Ende 17a der Elektrode 15 frei von einer dielektrischen Beschichtung. Weiter bevorzugt ist das distale Ende 17a der Elektrode 15 sowie ein sich daran anschließender Elektrodenschaft 17b, zumindest ein Abschnitt des Elektrodenschaftes 17b, welcher an das distale Ende 17a der Elektrode 15 angrenzt, frei von einer dielektrischen Beschichtung. Vorzugsweise ist nicht nur das distale Ende 17a der Elektrode 15, sondern auch ein sich daran anschließender Abschnitt des Elektrodenschaftes 17b frei von einer dielektrischen Ummantelung. Das System 10 arbeitet nicht nach dem Prinzip der dielektrischen Barriereentladung (dielectric barrier discharge, DBD). Das HF-Gerät 11 ist mit dem Instrument 14 verbunden, um die Elektrode 15 mit elektrischer Leistung zu speisen. Das Instrument 14 weist einen Gaskanal 16 auf, in welchem sich die Elektrode 15 längs erstreckt. Das distale Ende 17a der Elektrode 15 kann innerhalb des Gaskanals 16 angeordnet sein (wie dargestellt), die Elektrode 15 kann mit dem Gaskanal 16 abschließen oder die Elektrode 15 kann aus dem Gaskanal 16 hervorschauen. Der Innendurchmesser des distalen Endes des Gaskanals 16 ist bevorzugt zwischen einschließlich 0,5 mm und einschließlich 10 mm, besonders bevorzugt zwischen 0,8 mm und 3 mm, beispielsweise 2,4 mm. Die Gaszuführungseinrichtung 13 ist mit dem Gaskanal 16 fluidisch verbunden, um den Gaskanal 16 mit Gas, bevorzugt Inertgas, besonders bevorzugt Edelgas, beispielsweise Argon, zu beaufschlagen. Zu dem System 10 gehört eine flächige Neutralelektrode 12, die großflächig mit dem Körper 18 des Patienten, hier stark schematisch dargestellt, elektrisch leitfähig verbunden ist. Das Instrument 14 kann ein Instrument 14 sein, welches auch für die Argon-Plasma-Koagulation verwendet werden kann.

Durch Beaufschlagung des Instruments 14 mit HF-Spannung mittels des HF-Geräts 11 wird zwischen der Elektrode 15 und dem Körper 18 des Patienten ein physikalisches Plasma 19 gezündet. Die Oberfläche der Elektrode 15 hat galvanischen Kontakt zu dem Plasma 19. Der elektrische Stromkreis wird von der Elektrode 15, durch das Plasma 19, durch den Körper 18 des Patienten zur Neutralelektrode 12 zurück zum HF-Gerät 11 geschlossen. Dabei treten Elektronen aus der Elektrode 15 in dem Instrument 14 in das stromführende Plasma 19 ein oder umgekehrt. Das Plasma 19 hat galvanischen Kontakt zu dem Körper 18 des Patienten. Der elektrisch leitfähige Körper 18 des Patienten kann daher als zweite Elektrode des Systems 10 aufgefasst werden, die galvanischem Kontakt zum Plasma 19 an der zu behandelnden Gewebestelle 21 aufweist. In dem System 10 gemäß Figur 1a haben demnach im Einsatz zwei Elektroden 15, 21 galvanischen Plasmakontakt. Der Abstand von der ersten Elektrode 15 zu dem Gewebe 21 ist ohne zusätzliche Maßnahmen variabel und hängt von der Führung des Instruments 14 durch den Anwender ab. Das Plasma 19 dient als "Leitungsstück" für elektrischen Strom von der Elektrode 15 zum Körper 18 des Patienten. Der Körper 18 des Patienten stellt ein weiteres Leitungsstück für elektrischen Strom zwischen dem Plasma 19 und der Neutralelektrode 12 dar. Der durch die Gaszuführungseinrichtung 13 zugeführte Gasstrom 20, welcher zumindest teilweise in den Plasmazustand überführt ist, verdrängt zumindest zum Teil der Luft oder eines anderen an der Stelle die Atmosphäre bildendenden Gases (z.B. reiner Stickstoff) oder Gasgemischs zwischen der Elektrode 15 und der Gewebestelle. Das System 10 gemäß Figur 1a ist ein Beispiel eines Systems 10, bei dem die elektrische Stromflussrichtung mit der Plasmaströmungsrichtung zum Gewebe 21 übereinstimmt.

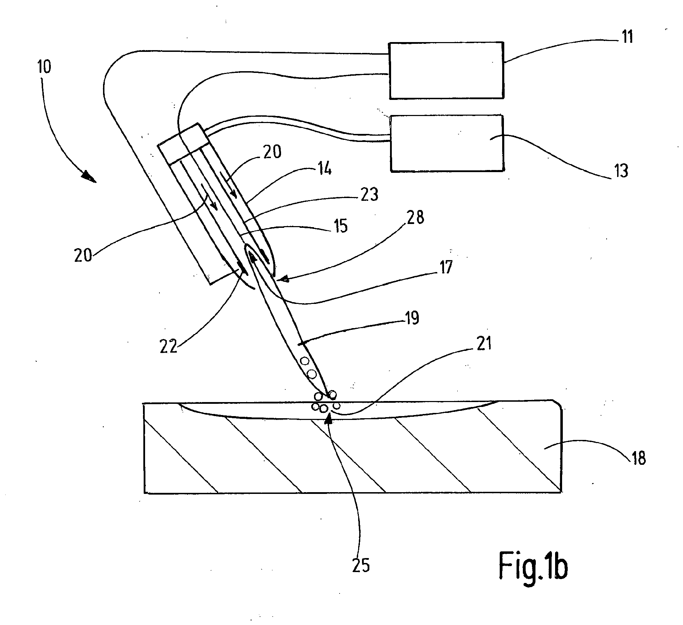

In Figur 1b ist ein Beispiel einer weiteren Ausführungsform eines erfindungsgemäßen Systems 10 dargestellt. Auch diese Ausführungsform des Systems 10 arbeitet nicht nach dem Prinzip der dielektrischen Entladung. Das System 10 arbeitet nach dem Jet-Prinzip. Das System 10 weist ein HF-Gerät 11, eine Gaszuführungseinrichtung 13, ein medizinisches Instrument 14 mit einer ersten Elektrode 15 und einer ringförmigen zweiten Elektrode 22 auf. Das HF-Gerät 11 ist mit dem Instrument 14 verbunden, um die erste Elektrode 15 mit elektrischer Spannung zu speisen. Das Instrument 14 weist eine Kapillare 23 aus einem elektrisch isolierenden Material auf, in welcher sich die erste Elektrode 15 längs erstreckt. Die zweite Elektrode 22 umschließt das Ende der Kapillare 23. Die Elektrodenspitze 17a der ersten Elektrode 15 ist innerhalb der Kapillare 23 angeordnet. Die erste Elektrode 15 und die zweite Elektrode 22 sind in festem Abstand zwischen einander angeordnet. Die erste Elektrode 15 und die zweite Elektrode 22 liegen einander nicht direkt gegenüber, sondern zwischen erster Elektrode 15 und zweiter Elektrode 22 ist das isolierende Material der Kapillare 23 angeordnet. Die Gaszuführungseinrichtung 13 ist mit der Kapillare 23 fluidisch verbunden, um die Kapillare 23 mit Gas, bevorzugt Inertgas, besonders bevorzugt Edelgas, beispielsweise Argon, zu speisen. Eine Neutralelektrode 12 ist in der Ausführungsform des Systems 10 gemäß Figur 1b nicht vorhanden. Bei gezündetem Plasma 19 wird der Stromkreis vielmehr über das Plasma 19 zwischen der ersten Elektrode 15 zu der zweiten Elektrode 22 geschlossen. Auf Grund des galvanischen Kontakts zwischen der ersten Elektrode 15 und dem Plasma 19 können Elektronen aus der ersten Elektrode 15 in das Plasma 19 eintreten oder umgekehrt. Die zweiten Elektrode 22 hat auf Grund der elektrisch isolierenden Kapillare 23 zwischen erster Elektrode 15 und zweiter Elektrode 22 keinen galvanischen Kontakt zu dem Plasma 19. Das Plasma 19 wird durch den der Kapillare kontinuierlich zugeführten Gasstrom 20 aus der Kapillare 23 zu der zu behandelnden Gewebestelle 21 ausgeblasen. Zudem verdrängt der Gasstrom bzw. Plasmastrom zumindest zum Teil die Luft oder eines anderen an der Stelle die Atmosphäre bildendenden Gases (z.B. reiner Stickstoff) oder Gasgemischs zwischen der Elektrode und der Gewebestelle.

Mittels des Plasmas 19 erzeugt mit dem System 10 gemäß erster Ausführungsform (Figur 1a) und mit dem Plasma 19 erzeugt mit dem System 10 gemäß zweiter Ausführungsform (Figur 1b) werden therapeutisch wirksame Spezies 25 (Atome, Moleküle, Ionen), insbesondere neutrale oder geladene Radikale, in der Gewebestelle 21 erzeugt und/oder in die Gewebestelle 21 eingetragen. Insbesondere das mit dem System 10 gemäß erster Ausführungsform (Figur 1a) erzeugte Plasma 19 führt zu einer besonders hohen Dichte an neutralen und geladenen Spezies 25, beispielsweise reaktiven Sauerstoffspezies und/oder reaktiven Stickstoffspezies in dem Plasma 19 und/oder in der zu behandelnden Gewebestelle 21. Die derart aktivierte Gewebestelle 21 bildet ein mittels des Plasmas aktiviertes Medium, wobei die Aktivierung zur Rückbildung beispielsweise von Neoplasien führt.

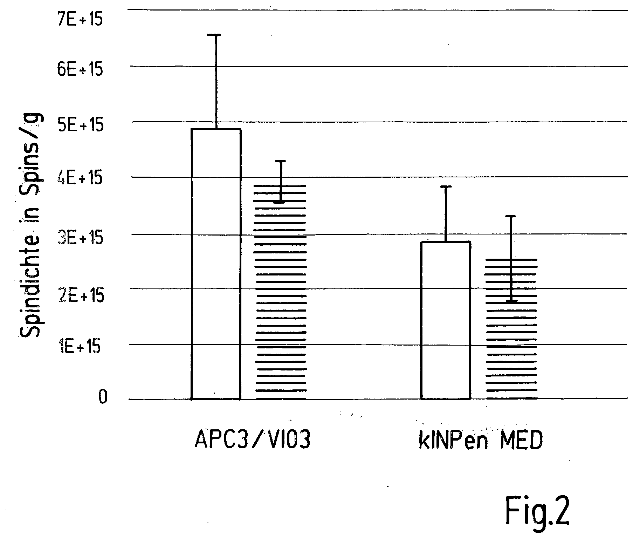

Die im Zusammenhang mit den Figuren 1 und 2 beschriebenen Ausführungsformen von Instrumenten 14 können verglichen mit Instrumenten 14, welche nach dem Prinzip der dielektrischen Barriereentladung arbeiten, einen schlankeren Aufbau aufweisen. Dies gilt insbesondere für Ausführungsformen wie im Zusammenhang mit Figur 1a beschrieben, da bei diesem nur eine Elektrode 15 am Instrument 14 vorhanden sein muss. Auch können die Instrumente 14, wie im Zusammenhang mit den Figuren 1 und 2 beschrieben, in größerem Abstand des distalen Instrumentenendes 28 über das Gewebe 21 geführt werden, was die Handhabung erleichtert. Ausführungsformen, wie im Zusammenhang mit Figur 1a erläutert, zeichnen sich gegenüber Ausführungsformen, wie im Zusammenhang mit Figur 1b erläutert, dadurch aus, dass in dem wenigstens einen Plasmakanal 19 zwischen der ersten Elektrode 15 und der Gewebestelle 21 des mit der Neutralelektrode 12 verbundenen Körpers Radikale nahe am Gewebe ständig neu erzeugt werden. Die ermöglicht auch besonders kurzlebigen radikalen Spezies zum Gewebe 21 zu gelangen. Bei Instrumenten 15, wie im Zusammenhang mit Figur 1b erläutert, findet die Plasmaerzeugung dagegen weiter entfernt von der Gewebestelle 21 statt und das Plasma 19 muss erst aus dem Instrument 15 ausgeblasen werden. Als Vorteil von Plasmen 19 erzeugt mittels eines Systems 10 gemäß Ausführungsformen, wie im Zusammenhang mit Figur 1a beschrieben, bei der Behandlung von hierin beschriebenen Krankheiten bzw. der Herstellung von aktivierten Medien zur Behandlung von hierin beschriebenen Krankheiten gegenüber Systemen 10 gemäß Ausführungsformen, wie im Zusammenhang mit Figur 1b beschrieben, wird eine höhere Anzahl an gebildeten Radikalen nach gleicher Einwirkungsdauer der Plasmen 19 auf menschliches oder tierisches Gewebe, insbesondere Epithel, oder anderes körpereigenes oder körperfremdes Medium gesehen. Die erhöhte Anzahl an gebildeten Radikalen bei gleicher Einwirkungsdauer der Plasmen 19 und gleichem Abstand zwischen jeweils dem distalen Instrumentenende 28 und der zu behandelnden Oberfläche 21 kann beispielsweise durch Ermittlung der Spindichte in behandelten Proben gleicher Menge mittels Elektronenspinresonanz (ESR)-Messung nachgewiesen werden, mit welcher ein Maß für die Anzahl der Radikale in den Proben erhalten werden kann. Als Proben kommen beispielsweise Proben menschlichen oder tierischen Gewebes oder Lösungen mit als Spinfallen (spin trap) bekanntem Material in Frage. Eine Lösung kann beispielsweise aus 0,1 Mol DMPO (5,5-Dimethyl-1-pyrrolidin-N-oxid), einem Beispiel einer Spinfalle, in entgastem DPBS⁺ (Zusammensetzung siehe unten) gebildet werden. Die Gewebeproben und/oder die Lösung können bei der Behandlung mit dem monopolaren Instrument 14 einer Ausführungsform des Systems 10 entsprechend Figur 1 beispielsweise auf der Neutralelektrode 12 angeordnet werden oder die Neutralelektrode 12 ist mit dem Probenbehälter oder - träger elektrisch leitend verbunden. Figur 2 zeigt nach Behandlung von präputialen Gewebeproben mit 10 Sekunden (flächig dunkle Balken) und 30 Sekunden (Balken aus übereinander angeordneten horizontalen Linien) Behandlungszeit ermittelte Spindichten in den Gewebeproben unter Verwendung der Geräteparameter 'preciseAPC' (gepulster Mode mit Wiederholrate von 10 ms, entsprechend 100 Hz Niederfrequenz, und einer Mittelfrequenz von 20 kHz), Effektstufe 1, 1,6 Liter/min Argon. Effektstufe 1 bedeutet, dass die mittlere Wirkleistung, mit welcher ein Bemessungswiderstand von 1000 Ohm beaufschlagt wird, wenn dieser mit dem Potential der Instrumentenelektrode 15 und dem Potential der Neutralelektrode 12 verbunden ist, maximal 2 W beträgt. Bei längerer Behandlungszeit (vergleiche 30s Behandlungszeit mit 10s Behandlungszeit) nimmt die erreichte Spindichte wieder ab. Die Fehlerbalken zeigen eine Standardabweichung an. Das verwendete System APC3/VIO3 ist ein Beispiel einer Ausführungsform des Systems 10 wie im Zusammenhang mit Figur 1a erläutert. Das verwendete System kINPen MED ist ein Beispiel einer Ausführungsform des System 10 wie im Zusammenhang mit Figur 1b erläutert. Das Funktionsschema der Plasmaerzeugung in diesem Gerät ist eine Quarzkapillare mit dem Innendurchmesser von 1,6 mm im Innern des Handgeräts, in der eine stiftförmige Radiofrequenz-Elektrode mit dem Außendurchmesser von 1 mm zentral eingebracht ist, und an der eine Hochfrequenzspannung (1,1 MHz, 2-6 kVpp) dauerhaft anliegt, welche das vorbeiströmende Gas mit einem für den Vergleichsversuch verwendeten Gasfluss von 4,1 l/min ionisiert. Der Abstand zu den Testmedien betrug stets 7 mm. Wie sich aus Figur 2 ergibt, ist nach Behandlung mit dem System APC3/VIO3 eine höhere Spindichte in einer Gewebeprobe zu verzeichnen als nach Behandlung einer vergleichbaren Gewebeprobe mit dem System kINPen MED. Die Wirksamkeit der Plasmen 19 bei der Behandlung einer oder mehrerer der hierin beschriebenen Krankheiten wird auf die mittels des Plasmas in dem Gewebe erzeugten oder in das Gewebe eingetragenen radikalen Spezies zurückgeführt.

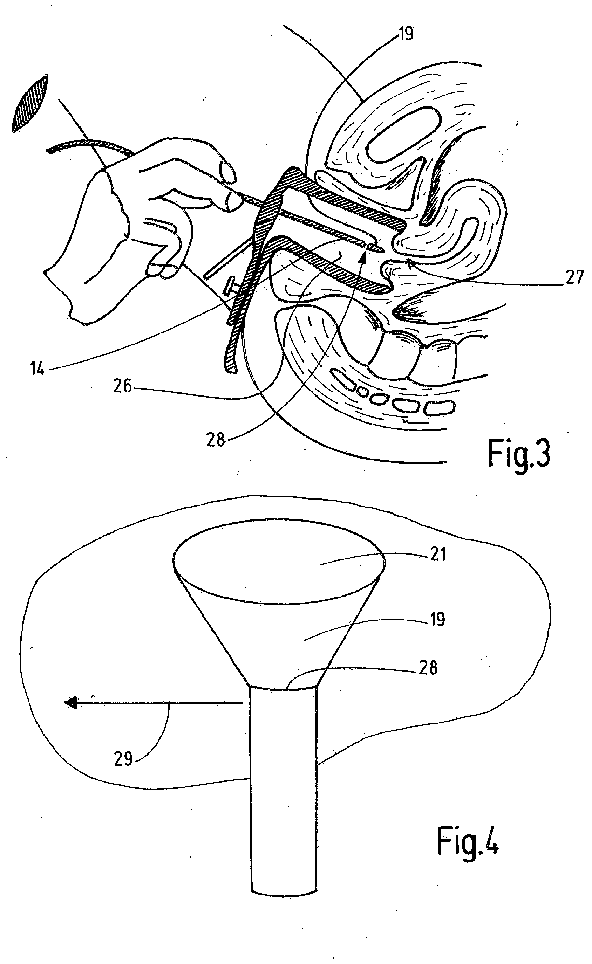

Figur 3 veranschaulicht die Verwendung von erfindungsgemäßem Plasma 19 zur Prävention von zervikalen intraepitelialen Neoplasien, insbesondere durch antivirale Therapie, z.B. gegen das humane Papilloma-Virus, zur Behandlung von zervikalen intraepitelialen Neoplasien aller Schweregrade, beispielsweise zervikale Carcinoma in situ Läsionen und/oder zur Behandlung von zervikalen invasiven Karzinomen beim Menschen mittels Plasma 19 erzeugt mittels des Systems 10 gemäß der ersten Ausführungsform oder des Systems 10 der zweiten Ausführungsform (Figuren 1, 2). Die Neutralelektrode 12 (in Figur 3 nicht dargestellt) muss bei der Behandlung mit dem System gemäß Figur 1a an geeigneter Stelle großflächigen Kontakt mit dem Körper 18 der Patientin haben. Das Instrument 14 wird durch die Vagina 26 bis an oder in die Cervix uteri 27 heran bzw. eingeführt und das Plasma 19 gezündet. Wie in Figur 4 veranschaulicht überstreicht das Plasma 19 durch beispielsweise händisches Bewegen (angedeutet durch den Pfeil 29) der Instrumentenspitze 28 (distales Ende des Instruments), vorzugsweise mit einer Mindestapplikationsgeschwindigkeit von z.B. 10 mm/s, die zu behandelnde Gewebestelle 21. Die Applikationsgeschwindigkeit ist die Geschwindigkeitskomponente der Instrumentenspitze 28 parallel zur Oberfläche der Gewebestelle 21. Trotz Herstellung eines warmen Plasmas kommt es durch das Bewegen bevorzugt nicht zu einer thermischen Schädigung der Oberfläche der Gewebestelle 21, insbesondere nicht zu einer Koagulation. Die bevorzugte Verwendung des Plasmas 19 wird daher als nicht-thermische Applikation bezeichnet. Um mit einer händisch erreichbaren und kontrollierbaren Applikationsgeschwindigkeit von bevorzug maximal 50 mm/s oder kleiner arbeiten zu können, so dass die Gewebetemperatur an der Gewebestelle 21, welche mit dem Plasma 19 überstrichen wird, kleiner oder gleich einer Grenztemperatur, beispielsweise 40°C, besonders bevorzugt kleiner oder gleich 37°C bleibt bzw. so dass eine thermische Gewebeschädigung, insbesondere Koagulation, durch die Plasmabehandlung ausbleibt, weist das Plasma 19 bevorzugt eine Temperatur von kleiner oder gleich einer Grenztemperatur auf, wobei die Grenztemperatur beispielsweise 150°C oder 120°C sein kann.

Der Gasfluss des Gases (Plasmagas) z.B. Argon dient dazu, eine geeignete, möglichst definierte, Mischatmosphäre zwischen distaler Instrumentenspitze und Gewebe zu schaffen, so dass eine Zündung des Plasmas 19 möglich ist. Bei zu niedrigem Gasfluss befindet sich zu wenig Plasmagas zwischen Elektrode 15 und Gewebe 21. Der Mindestgasfluss ist derjenige Gasfluss, der mindestens durch das Instrument 14 strömen muss, damit ein Plasma 19 zündet. Bei zu hohem Gasfluss wird durch Turbulenz zu viel Luft oder anderes Umgebungsgas oder -medium zugemischt, als dass ein Plasma 19 gezündet werden könnte. Der Höchstgasfluss ist derjenige Gasfluss, der höchstens durch das Instrument 14 strömen darf, damit ein Plasma 19 zündet. Eine reine Plasmagas-Atmosphäre kann mutmaßlich die Bildung von therapeutisch wirksamen Spezies erschweren. Daher liegt der gewählte Gasfluss des Gases (Plasmagas), z.B. Argon, zur Erzeugung des Plasmas 19 vorzugsweise in einem Bereich bei dem Mindestgasfluss oder in einem Bereich bei dem Höchstgasfluss. Der gewählte Gasfluss, der durch das Instrument 14 strömt, kann beispielsweise wenigstens so groß sein wie der Mindestgasfluss, aber kleiner als der Höchstgasfluss. Der Gasfluss an Plasmagas wird vorzugsweise in einem Bereich von einschließlich dem Mindestgasfluss bis einschließlich dem Gasfluss gewählt, der z.B. drei Mal größer ist als der Mindestgasfluss. Der Mindestgasfluss, und auch der Höchstgasfluss, können von mehreren Parametern und/oder Einstellungen abhängen. Der Mindestgasfluss kann insbesondere von einem gewünschten Behandlungsabstand zwischen Instrumentenspitze und Gewebeoberfläche abhängen. Für die Behandlung kann beispielsweise der Behandlungsabstand zwischen Instrumentenspitze und Gewebeoberfläche (z.B. 7 Millimeter) festgelegt und an Hand des festgelegten Behandlungsabstandes der Gasfluss gewählt werden, der durch das Instrument strömen soll. Bei einem Innendurchmesser des Gaskanals am distalen Ende von 2,4 mm beispielsweise kann der Gasfluss, beispielsweise bei einem Behandlungsabstand von 7 mm, bevorzugt in einem Bereich von einschließlich 1 l/min und einschließlich 3 l/min, besonders bevorzugt von einschließlich 1,3 l/min bis einschließlich 2,5 l/m, beispielsweise 1,6 l/min ± 20% sein. Bei kleinerem Innendurchmesser kann, bei ansonsten gleichen Parametern und Einstellungen, ein kleinerer Gasfluss, bei größerem Innendurchmesser, bei ansonsten gleichen Parametern und Einstellungen, ein größerer Gasfluss vorteilhaft sein.

Die Erfindung ist in Figur 3 anhand der Behandlung von zervikaler intraepithelialer Neoplasie einer Patientin über den transvaginalen Weg veranschaulicht. Allgemein kann die Applikation zur Behandlung von intraepithelialen Neoplasien unterschiedlicher Organsysteme von Säugern über den transvaginalen, oralen, parenteralen, laparoskopischen, intranasalen, intrabronchialen und rektalen Weg oder über mittels erfindungsgemäßem Plasma 19 aktivierten Medien oder Materialien (Medikamenten) erfolgen. Wenn das Plasma 19 zur Herstellung eines aktivierten körperfremden oder körpereigenen Mediums 21 verwendet wird, kann das Plasma 19 dadurch mittelbar zur Behandlung insbesondere einer oder mehrerer der in dieser Beschreibung genannten Krankheiten eingesetzt werden. Bei der Herstellung insbesondere aktivierten körperfremden Mediums kann die Neutralelektrode 12 von einem Träger oder Behälter (nicht dargestellt) des Mediums gebildet werden, wenn die Herstellung mit einem System 10 gemäß Ausführungsformen erfolgt, wie diese anhand der Figur 1a erläutert sind. Die nicht-thermische Applikation von atmosphärischem Argonplasma 19 eignet sich zur Behandlung von zervikalen intraepithelialen Neoplasien unterschiedlicher Pathogenese und zur Prophylaxe invasiver Karzinome. Intraepitheliale Neoplasien schließen hierbei auch intraepithelialen Neoplasien unterschiedlicher Organsysteme von Säugern, von z.B. Cervix Uteri, Os, Oesophagus, Gaster, Colon, Rektum und Peritoneum ein ohne darauf beschränkt zu sein. Das Einsatzspektrum des erfindungsgemäßen Plasmas 19 beinhaltet auch die prophylaktische Vorsorge von zervikalen und anderen intraepithelialen Neoplasien, Carcinoma in situ (CIS)-Läsionen und invasiven Karzinomen unterschiedlicher Pathogenese, in für die jeweilige Erkrankung prädispositionierten Säugern. Behandlung beinhaltet Verminderung der Erkrankung, Stopp oder Verzögerung der Progression, Induktion einer Remission von zervikalen und anderen intraepithelialen Neoplasien, CIS-Läsionen und invasiven Karzinomen unterschiedlicher Pathogenese in für die jeweilige Erkrankung prädispositionierten Säugern. Kombinationen mit anderen Verfahren, die nützlich sind für die Prävention oder die Behandlung von zervikalen und anderen intraepithelialen Neoplasien, CIS-Läsionen und invasiven Karzinomen unterschiedlicher Pathogenese sind möglich.

Der Nachweis des Effekts einer nicht-thermischen Applikation von atmosphärischem Plasma 19, insbesondere Argonplasma, zur Behandlung von intraepitelialen Neoplasien begründet sich auf standardisierten und kontrollierten Ex-vivo-, In-vitro- und In-vivo-Studien, deren Durchführung und erzielte Ergebnisse im Folgenden beschrieben sind. Für die nicht-thermische Applikation von atmosphärischem Argonplasma 19 im Rahmen der Studien wurde je ein VIO3/APC3-Generator (ERBE Elektromedizin, Tübingen), ein Beispiel eines HF-Geräts 11 und einer Gaszuführungseinrichtung 13, verwendet. Das HF-Gerät 11 erzeugt eine Wechselspannung mit einer Frequenz von 350 kHz. Als ein Beispiel für ein Instrument 14 wurde eine FiAPC-Sonde (ERBE Elektromedizin, Tübingen) mit einem Außendurchmesser von 3,2 mm und einem Innendurchmesser des distalen Endes des Gaskanals 16 von 2,4 mm verwendet. Die Sonde verfügt über ein Edelstahlplättchen als Elektrode mit einer Spitze am distalen Ende. Es wurden folgende Parameter verwendet: preciseAPC (gepulster Mode mit Wiederholrate von 10 ms, entsprechend 100 Hz Niederfrequenz, und einer Mittelfrequenz von 20 kHz), Effektstufe 1, 1,6 Liter/min Argon. Effektstufe 1 bedeutet, dass die mittlere Wirkleistung, mit welcher ein Bemessungswiderstand von 1000 Ohm beaufschlagt wird, wenn dieser mit dem Potential der Instrumentenelektrode 15 und dem Potential der Neutralelektrode 12 verbunden ist, maximal 2 W beträgt.

Die Untersuchung der Temperaturentwicklung bei Applikation von atmosphärischem Argonplasma 19 wurde ex-vivo durchgeführt. Die Untersuchung der Effekte auf neoplastischen Zellen anhand von Zervixkarzinomzelllinien wurde in-vitro durchgeführt. Die Untersuchung der Effekte auf zervikale intraepithelialen Neoplasien Grad I und II wurde in-vivo durchgeführt.

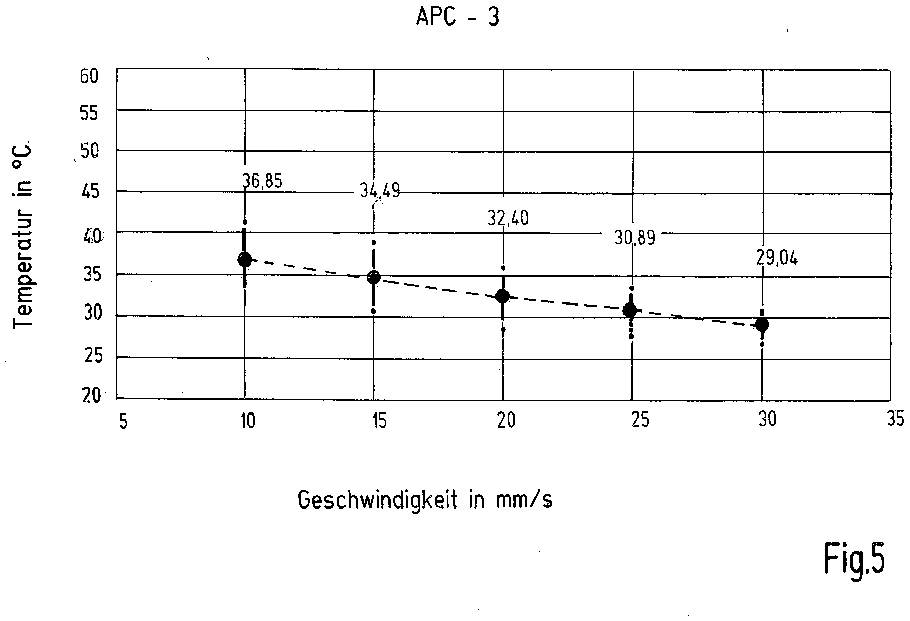

Die Möglichkeit zur nicht-thermischen Applikation des atmosphärischen Argonplasmas 19 gründet sich auf Ex-vivo-Gewebe-Untersuchungen mittels standardisierter Infrarot-Thermografie. Hierbei wurden Hydrogel-Proben in unterschiedlichen Applikationsgeschwindigkeiten mit atmosphärischem Argonplasma behandelt. Applikationsgeschwindigkeiten von größer 10 mm/s zeigten hierbei durchschnittlich stabile und unkritische Gewebetemperaturen kleiner 37° Celsius. Figur 5 zeigt die Gewebeoberflächentemperatur von Hydrogel während einer Applikation von atmosphärischem Argonplasma 19 mit unterschiedlichen Applikationsgeschwindigkeiten Ex-vivo. Die Ergebnisse sind dargestellt als Einzelmesspunkte (kleinere, dunkle Punkte) und die durchschnittliche Absoluttemperatur (größere, helle Punkte). Ein Abstand von 7 mm zwischen Instrumentenspitze und Oberfläche der Gewebeprobe wurde konstant gehalten. Die Instrumentenspitze war während den Messungen senkrecht zu der Oberfläche der Hydrogel-Proben ausgerichtet. Um für die Behandlung an der Oberfläche von menschlichem oder tierischem Gewebe aussagefähige thermografische Testmessungen durchführen zu können, wurden Hydrogelproben aus einer 12,5 % Stammlösung von Gelatine Typ B und Dulbecco's Phosphate Buffered Saline (DPBS⁺) hergestellt. DPBS⁺ ist eine wasserbasierte Salzlösung. Sie ist isotonisch, so dass ihre Ionenkonzentration der des menschlichen Körpers entspricht. Sie weist eine pH-Wert von 7,0 bis 7,3 auf. Zur Herstellung der Hydrogelproben wurde die Stammlösung chemisch mit 1-Ethyl-3-(3-dimethylaminopropyl)carbodiimid (EDC) verhärtet und in einer Polyactideform (70 x 20 x 5 mm) für zwei Stunden, bei 37°C und 100 rpm, in einen Schüttler gestellt. Nach einem Waschvorgang mit reinem DPBS⁺ waren die Hydrogel-Proben bereit, für die Testmessungen verwendet zu werden.

Die Ergebnisse der standardisierten Infrarot-Thermografie zeigen die Möglichkeit einer nicht-thermischen Applikation des erfindungsgemäßen atmosphärischem Argonplasmas 19 mit einer Gewebetemperatur von <37° Celsius unter Verwendung der beschriebenen Geräte und Einstellungen ab einer Applikationsgeschwindigkeit von ≥ 10 mm/s.

Der Effekt einer nicht-thermischen Applikation des erfindungsgemäßen, atmosphärischen Argonplasmas 19 auf eine Zervixkarzinomzelllinie (SiHa) wurde in-vitro untersucht. Die Ergebnisse wurden durch standardisierte Zellzahlmessungen mit einem CASY cell counter and analyzer (Roche) nach nicht-thermischer Applikation von erfindungsgemäßem atmosphärischem Argonplasma 19 mit Applikationsgeschwindigkeiten ≥ 10 mm/s und unterschiedlichen Dosierungen von 0 Sekunden bis 120 Sekunden und nach mehreren Inkubationszeiten von 24 Stunden bis 120 Stunden gewonnen. Die Plasmabehandlung wurde in handelsüblichem Debulko's modified eagle's medium (DMEM) mit 10% Fetal Calf Serum, 1% Penicillin/Streptomycin durchgeführt. Die Inkubation erfolgte unter humidifizierter Atmosphäre (37 °C, 5% CO₂, pH 7,4).

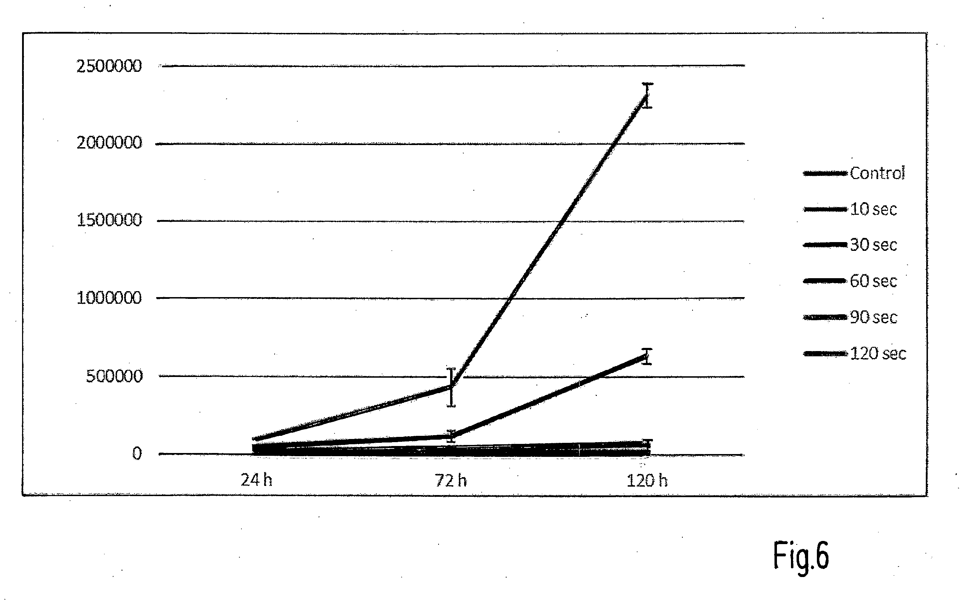

Figur 6 zeigt die Ergebnisse einer nicht-thermischen Applikation von atmosphärischem Argonplasma 19 auf Zervixkarzinomzelllinien in-vitro. Dargestellt sind die durchschnittlichen absoluten Zellzahlen aus N=6 unabhängig durchgeführten Messungen nach 24, 72 und 120 Stunden. Die Fehlerbalken zeigen +/- eine Standardabweichung an.

Die In-vitro-Ergebnisse der standardisierten Zellzahlmessungen mit einem CASY cell counter and analyzer (Roche) nach nicht-thermischer Applikation von erfindungsgemäßem atmosphärischem Argonplasma 19 mit Applikationsgeschwindigkeiten ≥ 10 mm/s und mit unterschiedlichen Dosierungen von 0 Sekunden bis 120 Sekunden zeigten eine dosisabhängige und zur Dosis proportionale Inhibition der Proliferation von Zervixkarzinomzelllinien (SiHa-Zellen) innerhalb der Inkubationszeiten von 24 Stunden bis 120 Stunden.

Der Effekt einer nicht-thermischen Applikation von erfindungsgemäßem atmosphärischem Argonplasma 19 auf CIN der Grade I und II wurde in-vivo untersucht. Die Fähigkeit einer nicht-thermischen Applikation von erfindungsgemäßem atmosphärischem Argonplasma 19, bei histologisch gesicherter CIN Grad I/Grad II eine Remission zu bewirken, wurde durch standardisierte zytologische Untersuchungen (Pap-Zytologie) nach 2 Wochen, 3 Monaten und 6 Monaten und histologische Untersuchungen (Fremdbefundung durch Institut für Pathologie, Tübingen) nach 3 und 6 Monaten analysiert. Im Folgenden wird das Verfahren skizziert. Patientinnen mit histologisch gesicherter CIN Grad I oder Grad II erhielten vor der erfindungsgemäßen nicht-thermischen Behandlung mit atmosphärischem Argonplasma 19 eine klinisch-kolposkopische Untersuchung mit Essig-Iod-Probe, um die Läsion darzustellen. Danach erfolgte unter kolposkopischer Sicht einmalig eine nicht-thermische Applikation von atmosphärischem Argonplasma 19 mit Applikationsgeschwindigkeiten ≥ 10 mm/s und in Abhängigkeit von der Größe der Läsion mit einer minimalen Dosierung von ≥ 4 unmittelbar aufeinander folgenden Applikationszyklen von jeweils 30 Sekunden. Als Geräteeinstellungen wurden folgende Parameter gewählt: preciseAPC, Effektstufe 1, 1,6 Liter/min Argon. Es wurde keine lokale und allgemeine Sedierung und Analgesie verwendet.

Eingeschlossen wurden Patientinnen > 18 Jahre der Universitäts-Frauenklinik Tübingen mit gesicherter CIN Grad I oder Grad II, die vorab über den Befund und mögliche therapeutische Strategien aufgeklärt wurden. Die Patientinnen wurden prospektiv und nicht randomisiert rekrutiert. Als Einschlusskriterien für die Plasmabehandlung galten: Alter > 18 Jahre, histologisch gesicherte CIN Grad I oder Grad II, sicher beurteilbare Transformationszonen der Portio und Randbegrenzungen der Läsionen, schriftliches Einverständnis zur Teilnahme an der Studie nach Aufklärung. Als Ausschlusskriterien für die Plasmabehandlung galten: Histologisch gesicherte CIN III, nicht vollständig einsehbare Transformationszone, Anhalt für eine invasive Erkrankung, zu erwartende fehlende Compliance der Patientin oder Unfähigkeit der Patientin, den Sinn und Zweck der klinischen Prüfung zu verstehen, schwerwiegende Cardio-vaskuläre Erkrankungen, Wunsch nach einer klassischen Therapiemethode, fehlende Patienteneinwilligung. Als primärer Endpunkt wurde eine histologische Komplettremission der CIN Grad I oder Grad II definiert. Als sekundäre Endpunkte wurden eine partielle histologische Remission der CIN Grad I oder Grad II, verminderte HPV-Viruslast, Schmerzen und Lebensqualität, Gewebetoleranz und Verträglichkeit der Plasmabehandlung definiert.

Die folgende Tabelle zeigt die histopathologischen Ergebnisse, die erzielt wurden.

| CIN I/II vor Behandlung | Histologie 3 mo | Histologie 6 mo |
|---|---|---|
| 43 | 35 von 43 (82 %) | 25 von 27 (93 %) |

Diese Ergebnisse zeigen, dass die nicht-thermische Applikation von atmosphärischem Argonplasma 19 mit Applikationsgeschwindigkeiten ≥ 10 mm/s und einer Dosierung von ≥ 4 unmittelbar aufeinander folgenden Applikationszyklen von jeweils 30 s mit einer Remissionsrate der CIN I und II Läsionen von 82% innerhalb von 3 Monaten einhergehen. Nach 6 Monaten sind 93% der gesundeten Läsionen stabil und weiterhin histologisch unauffällig. Beispielsweise für die CIN II sind in der Literatur durchschnittliche Spontanremissionsraten zwischen 40 und 50% beschrieben. Verglichen dazu sind die Remissionsraten nach nicht-thermischer Applikation von atmosphärischem Argonplasma 19 signifikant höher.

Die folgende Tabelle zeigt die virologischen Ergebnisse, die mit dem System und den genannten Einstellungen erzielt wurden.

| High risk HPV positiv vor Behandlung | High risk HPV positiv 6 mo |
|---|---|
| 72 % | 11 % |

Diese Ergebnisse zeigen, dass die nicht-thermische Applikation von atmosphärischem Argonplasma 19 mit Applikationsgeschwindigkeiten ≥ 10 mm/s und einer Dosierung von ≥ 4 unmittelbar aufeinander folgenden Applikationszyklen von jeweils 30 s mit einer Remissionsrate der High risk HPV Possitivität um 60% innerhalb von 6 Monaten einhergehen.

Die Ergebnisse der standardisierten In-vitro- und In-vivo-Untersuchungen zeigen, dass die nicht-thermische Applikation von atmosphärischem Argonplasma 19 ein nützliches Verfahren in der Behandlung insbesondere von CIN-Läsionen ist.

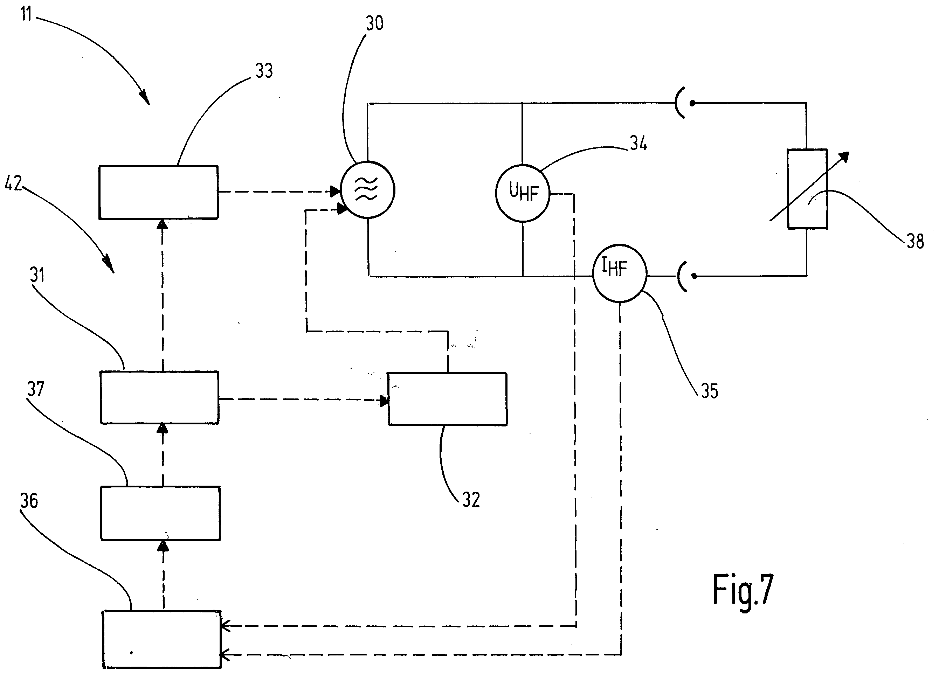

Figur 7 veranschaulicht ein HF-Gerät 11, welches in einem erfindungsgemäßen System 10, beispielsweise gemäß Figuren 1a und 1b eingesetzt werden kann. Das HF-Gerät 11 weist einen HF-Generator 30, eine Steuereinrichtung 31, eine Modulationseinrichtung 32 und ein Leistungsnetzteil 33 auf. Das System 10 kann eine erste Detektoreinrichtung 34 zur Bestimmung einer Spannung, eine zweite Detektoreinrichtung 35 zur Bestimmung eines Stroms, eine Signalauswerteeinrichtung 36 und eine Vergleichseinrichtung 37 aufweisen. Veranschaulicht ist außerdem eine Lastimpedanz 38, in welche im Falle des Systems 10 gemäß Figur 1a die Impedanz des Plasmas 19 und des Körpers 18 zwischen Plasma 19 und Neutralelektrode 12 und im Falle des Systems 10 gemäß Figur 1b die Impedanz des Plasmas 19 eingeht.

Der HF-Generator 30 ist dazu ausgebildet eine HF-Spannung zu generieren, welche eine HF-Frequenz, beispielsweise von mindestens 100 kHz, vorzugsweise zwischen einschließlich 200 kHz bis einschließlich 16 MHz, besonders bevorzugt zwischen 300 kHz und 500 kHz, beispielsweise 350 kHz aufweisen kann.

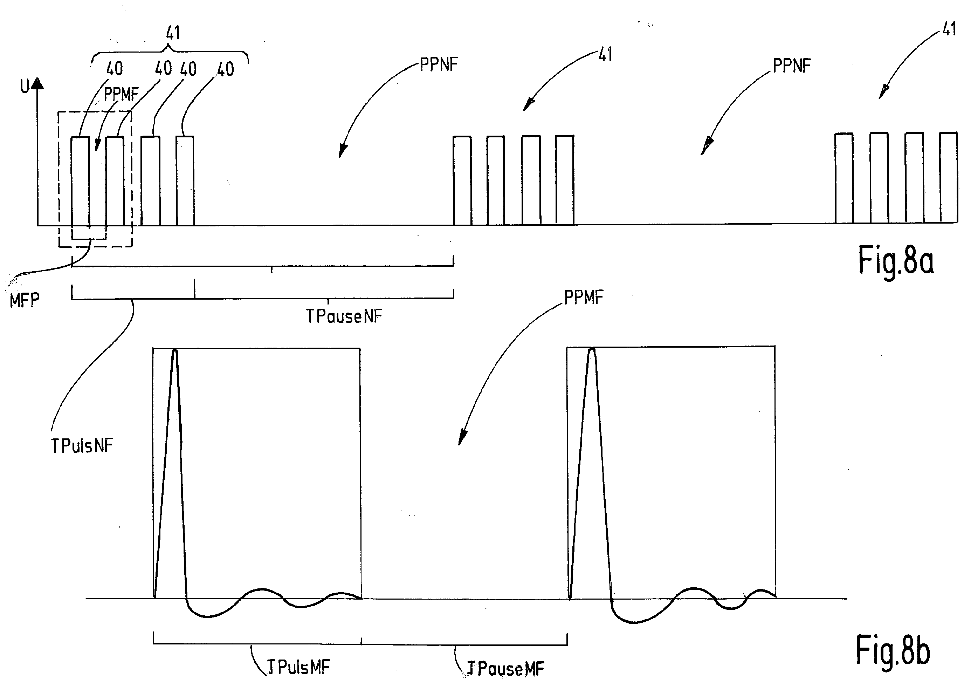

Figur 8a zeigt schematisch und beispielhaft eine modulierte Hochfrequenzausgangwechselspannung U des HF-Generators 30 im Zeitverlauf. Figur 8b zeigt einen Ausschnitt aus Figur 8a.

Die Wechselspannung U ist mittels der Modulationseinrichtung 32 bevorzugt mit einer Mittelfrequenz (MF) und/oder einer Niederfrequenz (NF) moduliert. Die Mittelfrequenz kann beispielsweise zwischen einschließlich 5 kHz und einschließlich 100 kHz, besonders bevorzugt zwischen einschließlich 10 kHz und einschließlich 50 kHz, beispielsweise 20 kHz betragen. Die Pulslänge TPulsMF der Mittelfrequenzpulse 40 beträgt vorzugsweise wenigstens eine oder mehrere HF-Perioden.

Die Niederfrequenz beträgt beispielsweise zwischen einschließlich 0,5 Hz und einschließlich 200 Hz, vorzugsweise zwischen einschließlich 20 Hz und einschließlich 150 Hz, insbesondere 100 Hz. Die Pulslänge TPulsNF der Niederfrequenzpulspakete 41 beträgt vorzugsweise mindestens eine Mittelfrequenzperiode MFP. Vorzugsweise zwischen einschließlich einer Mittelfrequenzperiode und 50 Mittelfrequenzperioden, beispielsweise 20 Mittelfrequenzperioden.

Die Modulation mit der Mittelfrequenz und/oder der Niederfrequenz kann dazu genutzt werden, z.B. bei vorgegebener Mindestspitzenspannung, die Ausgangsleistung gegenüber einer ungepulsten Wechselspannung zu reduzieren. Die Modulation oder Pulsung mit der Niederfrequenz führt zu einem Erlöschen des Plasmas 19 in den Pulspausen PPNF der Niederfrequenz und somit zu einem Ablösen des Plasmas 19 von der Gewebestelle 21. Dadurch bleibt das Plasma 19 trotz Bewegen der Spitze 28 des Instruments 14 längs parallel mit Abstand zur Gewebestelle 21 nicht an einer Gewebestelle 21 hängen. Vielmehr kann die zu behandelnde Gewebestelle 21 mit dem Plasma 19 gleichmäßig überstrichen werden.

In dem vorstehend angesprochenen Mode preciseAPC, Effektstufe 1 des HF-Geräts APC3/VIO3 (einem Beispiel für das System gemäß Figur 1a) beispielsweise, welcher zur Behandlung der Patientinnen verwendet wurde, sind die Mittelfrequenz MF und die Niederfrequenz NF fest vorgegeben (MF=20 kHz und NF=100 Hz), so dass sich bei Betrieb an einem festen Bemessungswiderstand ohne Plasmastrecke die für die jeweilige Effektstufe vorgesehene mittlere Leistung ergibt. Die Wahl der Effektstufe (1 bis 10) beeinflusst die dann feste Länge TPulsNF jedes der Niederfrequenzpulse. Wenn der Anwender die Instrumentenspitze 28 benachbart zu der Gewebestelle 21 hält und die Plasmageneration aktiviert, wählt das HF-Gerät 11 unabhängig von der Effektstufe die Ausgangsspannung so hoch, dass das Plasma 19 gezündet wird. In dem angesprochenen Mode preciseAPC, beispielsweise Effektstufe 1, des Systems VIO3 als ein Beispiel für das System 10, ist dieses dazu eingerichtet, zu Beginn eines Niederfrequenzpulses, welcher ein Paket aus mehreren Pulsen der Mittelfrequenz ist, die Spitzenausgangsspannung des HF-Geräts 11 so lange zu erhöhen bis entweder eine Obergrenze erreicht ist oder ein Plasma gezündet wird. Die Obergrenze beträgt beispielsweise zwischen einschließlich 2 kV und 6 kV, bevorzugt zwischen 4 kV und 5 kV, bei dem System mit VIO3 beispielsweise 4,7 kV. Das System 10 ist weiter dazu eingerichtet, dass bei von dem System 10 erkannter Zündung die Ausgangsspannung des Leistungsnetzteils 33 des HF-Geräts 11 abgesenkt wird. Insbesondere wird die Ausgangsspannung des Leistungsnetzteils 33 auf einen Sollwert geregelt, der kleiner ist als der für die Zündung erforderliche Wert. Dadurch wird eine Verminderung der Spitzenausgangsspannung des HF-Generators 30 erreicht, so dass zu Beginn des folgenden Niederfrequenzpulses die Spitzenspannung geringer ist als zum Zeitpunkt der Plasmazündung im Niederfrequenzpulspaket zuvor. Dadurch kann mit dem System VIO3 die Erzeugung eines zu intensiven Plasmas vermieden werden, das zu einem zu starken thermischen Effekt, insbesondere zu einer thermischen Gewebeschädigung führen könnte.

Jedoch ist die Zündspannung von dem Abstand zum Gewebe 21 abhängig, daneben auch beispielsweise von Eigenschaften der Elektrode 15 (z.B. Geometrie und/oder Material), verwendetem Gas, Eigenschaften des Gewebes und der Umgebung. Die Abstandsabhängigkeit bedeutet, dass die mittlere Ausgangsleistung des HF-Geräts 11 bei gleicher Effektstufe 1 von dem Abstand der Elektrodenspitze 17a vom Gewebe 21 abhängig ist. Beispielsweise kann die mittlere Ausgangswirkleistung des HF-Geräts 11 für eine bestimmte Effektstufe, z.B. Effektstufe 1, bei 3 mm Abstand ca. 1,5 W, bei 12 mm Abstand ca. 5 W, betragen. Dies stellt besondere Anforderungen an die Führung der Instrumentenspitze 28 über das Gewebe 21 durch den Anwender in möglichst immer gleichem Abstand und konstanter Geschwindigkeit, um einen gleichmäßigen Gewebeeffekt zu erzielen. Denn beim Bewegen über das Gewebe 21 kommt es durch die Modulation mit der Niederfrequenz zum wiederholten Zünden zu Beginn jedes Pulspaketes 41 der Niederfrequenz bei potentiell unterschiedlichen Abständen der Elektrodenspitze 17a zum Gewebe 21.

Erfindungsgemäß wird daher ein System 10 vorgeschlagen (dieses ist ebenfalls mittels Figur 7 veranschaulicht), welches eine Vorrichtung 42 zur Regelung der Ausgangsleistung, beispielsweise der Ausgangswirkleistung oder der Ausgangsscheinleistung, des HF-Geräts aufweist. Das System 10 kann eine Steuerung zur Verminderung der Spitzenausgangsspannung aufweisen wie vorstehend im Zusammenhang mit der Erläuterung zu dem System VIO3 dargestellt.

Dazu weist die Vorrichtung 42 eine Einrichtung zur Ermittlung der Ausgangswirkleistung auf. Beispielsweise kann die Vorrichtung dazu eingerichtet sein, die Ausgangsleistung des Leistungsnetzteils 33 zu ermitteln und mittels eines bekannten Wirkungsgrades des HF-Generators 30 die Ausgangswirkleistung des HF-Generators 30 ermitteln. Alternativ oder zusätzlich kann die Vorrichtung 42, wie in Figur 7 veranschaulicht, mittels der ersten Detektionseinrichtung 34 dazu eingerichtet sein, die Ausgangsspannung des HF-Generators 30 zu ermitteln und mittels der zweiten Detektionseinrichtung 35 den Ausgangsstrom des HF-Generators 30 zu ermitteln. Mittels der Signalauswerteeinrichtung 36 kann die Vorrichtung 42 dazu eingerichtet sein, aus den Messwerten der ersten Detektionseinrichtung 34 und der zweiten Detektionseinrichtung 35 die Ausgangsleistung des HF-Generators 30 zu bestimmen.

Die Ermittlung der Ausgangsleistung und die Anpassung der Modulation kann kontinuierlich oder in regelmäßigen oder unregelmäßigen zeitlichen Abständen erfolgen. Die Ausgangsleistung kann beispielsweise durch Mittelung des Produkts der in Phasenbeziehung gesetzten Spannung und Strom über eine oder mehrere Perioden, z.B. eine oder mehrere Mittelfrequenzperioden und/oder Niederfrequenzperioden, ermittelt werden. Die Ermittlung der Ausgangsleistung erfolgt dann vorzugsweise in der anschließenden Pulspause, insbesondere Pulspause PPNF der Niederfrequenz oder Pulspause der Mittelfrequenz PPMF. Die Ermittlung kann beispielsweise regelmäßig in den Pulspausen PPNF der Niederfrequenz erfolgen. Insbesondere kann die Ausgangsleistung über eine oder mehrere Mittelfrequenzperioden bis zum Ende eines Niederfrequenzpulspakets gemittelt werden und somit der Mittelwert der Ausgangsleistung als Ist-Wert der Ausgangsleistung nach Ende des Niederfrequenzpulspakets ermittelt werden.

Die Vorrichtung 42 kann dazu eingerichtet sein, mittels der Vergleichseinrichtung 37 die bestimmte Ausgangsleistung (Ist-Wert), z.B. die Ausgangswirkleistung, mit einem Soll-Wert der Ausgangsleistung, z.B. der Ausgangswirkleistung, zu vergleichen und bei Abweichen des Ist-Wertes von dem Soll-Wert mittels der Steuereinrichtung 31 dazu eingerichtet sein, eine oder mehrere Veränderungen vorzunehmen, um den Ist-Wert dem Soll-Wert anzunähern.

Es wäre zusätzlich oder alternativ grundsätzlich möglich zur Regelung den Spitzenwert der Ausgangsspannung des HF-Generators 30 abhängig von der ermittelten Abweichung des Ist-Wertes von dem Soll-Wert zu verändern. Dies erfordert jedoch bei sehr kurzen Pulspaketen 41 der Niederfrequenz eine hohe Regelgeschwindigkeit, die nur schwer zu realisieren ist. Außerdem kann eine verringerte Spitzenspannung zu einem stark verschlechterten Zündverhalten führen. Deshalb wird bevorzugt von einer Veränderung des Spitzenwerts der Ausgangsspannung zur Regelung abgesehen und stattdessen die Modulation der HF-Frequenz in Abhängigkeit von der Abweichung von Ist-Wert zum Soll-Wert der Ausgangsleistung, insbesondere Ausgangswirkleistung, verändert. Grundsätzlich ist alternativ eine Regelung möglich, bei welcher sowohl der Spitzenwert der Ausgangsspannung verändert wird, so dass dieser oberhalb eines Mindestwertes ist, und zusätzlich oder alternativ die Modulation verändert wird.

Mit der Leistungsregelung durch Anpassung der Modulation ist in Ausführungsformen eine Erfassung des Ist-Wertes der Ausgangsleistung in einem Pulspaket der Niederfrequenz und eine Anpassung der Modulation derart rasch möglich, dass der Soll-Wert der Ausgangsleistung innerhalb desselben Pulspaketes oder innerhalb des oder zu Anfang des ersten nachfolgenden Pulspaketes der Niederfrequenz erreicht wird. Die Veränderung der Modulation kann insbesondere die Niederfrequenzmodulation und/oder die Mittelfrequenzmodulation beinhalten. Beispielsweise kann das PulsPause-Verhältnis der Niederfrequenzpulspakete 41 verändert werden durch Verändern der Pulslänge TPulsNF, Verändern der Pausenlänge TPauseNF und/oder Verändern der Niederfrequenzperiode. Alternativ oder zusätzlich kann das Puls-Pause-Verhältnis der Mittelfrequenzpulse 40 verändert werden durch Verändern der Pulslänge TPulsMF, Verändern der Pausenlänge TPauseMF und/oder Verändern der Mittelfrequenzperiode.

Mit dem System 10 mit Vorrichtung 42 zur Regelung der Ausgangsleistung, beispielsweise der Ausgangswirkleistung, kann ein Plasma 19 mit konstanter Ausgangsleistung des HF-Generators unabhängig von den Zündbedingungen, insbesondere Abstand zum Gewebe 21 erzeugt werden, was die Führung des Instruments 14 zur effektiven, nicht thermischen Behandlung, wie diese beispielsweise in Figuren 3 und 4 dargestellt ist, erheblich vereinfacht. Ein System 10 mit einer Vorrichtung 42 zur Regelung der Ausgangsleistung bzw. ein damit erzeugtes Plasma 19 kann vorteilhaft insbesondere zur Behandlung einer oder mehrerer der vorstehend genannten Krankheiten verwendet werden. Allgemein kann eine System 10 mit einer Leistungsregelung wie im Zusammenhang mit der Vorrichtung 42 beschrieben vorteilhaft überall da eingesetzt werden, wo ein abstandsunabhängig reproduzierbarer Gewebeeffekt gewünscht wird. Durch die Leistungsregelung kann ein Gewebeeffekt besonders fein reguliert werden. Ein Einsatzfeld kann beispielsweise die nicht-thermische Wundbehandlung sein, wo eine thermische Schädigung des Wundgewebes vermieden werden soll.

Erfindungsgemäß wird insbesondere ein Plasma 19 zur Prävention von intraepithelialen Neoplasien, insbesondere durch antivirale Therapie, z.B. gegen das humane Papillomavirus, zur Behandlung von intraepithelialien Neoplasien aller Schweregrade, beispielsweise Carcinoma in situ Läsionen, und/oder zur Behandlung von invasiven epithelial erreichbaren Karzinomen geschaffen, wobei das Plasma erzeugt ist mit einem System 10 zur Erzeugung eines Plasmas 19, bevorzugt eines Argon-Plasmas, mit einem medizinischen Instrument 14 mit wenigstens einer Elektrode 15 mit galvanischem Kontakt zu dem Plasma 19, einem HF-Gerät 11 zur Bereitstellung einer Wechselspannung zur Speisung des Instruments 14 mit elektrischer Leistung und einer Gaszuführungseinrichtung 13, welche eingestellt ist, um das Instrument 14 mit Gas, insbesondere Argon, zu speisen.

**Bezugszeichenliste:**

| | |
|---|---|
| 10 | System |
| 11 | HF-Gerät |
| 12 | Neutralelektrode |
| 13 | Gaszuführungseinrichtung |
| 14 | medizinisches Instrument |
| 15 | Elektrode, erste Elektrode |
| 16 | Gaskanal |
| 17a | distales Ende der Elektrode |
| 17b | Elektrodenschaft |
| 18 | Körper |
| 19 | Plasma |
| 20 | Gasströmung |
| 21 | Gewebestelle/Medium |
| 22 | zweite Elektrode |
| 23 | Kapillare |
| 25 | Spezies |
| 26 | Vagina |
| 27 | Cervix uteri |
| 28 | Instrumentenspitze |
| 29 | Pfeil |
| 30 | HF-Generator |
| 31 | Steuereinrichtung |
| 32 | Modulationseinrichtung |
| 33 | Leistungsnetzteil |
| 34 | Erste Detektoreinrichtung |
| 35 | Zweite Detektoreinrichtung |
| 36 | Signalauswerteeinrichtung |
| 37 | Vergleichseinrichtung |
| 38 | Lastimpedanz |
| 40 | Mittelfrequenzpuls |
| 41 | Niederfrequenzpulspakete |
| 42 | Vorrichtung |
| TPulsMF | Pulslänge Mittelfrequenz |
| TPulsNF | Pulslänge Niederfrequenz |
| MFP | Mittelfrequenzperiode |
| PPNF | Pulspause Niederfrequenz |
| PPMF | Pulspause Mittelfrequenz |
| TPauseNF | Pausenlänge Niederfrequenz |
| TPauseMF | Pausenlänge Mittelfrequenz |

## Patentansprüche

1. Plasma (19) zur Prävention von intraepithelialen Neoplasien, insbesondere durch antivirale Therapie, z.B. gegen das humane Papillomavirus, zur Behandlung von intraepithelialien Neoplasien aller Schweregrade, beispielsweise Carcinoma in situ Läsionen, und/oder zur Behandlung von invasiven epithelial erreichbaren Karzinomen, wobei das Plasma erzeugt ist mit einem System (10) zur Erzeugung eines Plasmas (19), bevorzugt eines Argon-Plasmas, mit einem medizinischen Instrument (14) mit wenigstens einer Elektrode (15) mit galvanischem Kontakt zu dem Plasma (19), einem HF-Gerät (11) zur Bereitstellung einer Wechselspannung zur Speisung des Instruments (14) mit elektrischer Leistung und einer Gaszuführungseinrichtung (13), welche eingestellt ist, um das Instrument (14) mit Gas, insbesondere Argon, zu speisen.

2. Plasma (19) nach Anspruch 1 zur Behandlung von intraepithelialen Neoplasien wenigstens eines der folgenden Organsysteme von Säugern: Zervix uteri, Os, Oesophagus, Gaster, Colon, Rektum und Peritoneum.

3. Plasma (19) nach einem der vorstehenden Ansprüche zur Behandlung von intraepithelialen Neoplasien, insbesondere von zervikalen intraepithelialen Neoplasien, des Grades I bis III, beispielsweise des Grades I bis II, insbesondere des Grades II.

4. Plasma (19) nach einem der vorstehenden Ansprüche, wobei das System (10) eine Neutralelektrode (12) aufweist, wobei die Neutralelektrode (12) an dem Körper (18) eines Patienten angeordnet ist, um den Stromkreis von dem HF-Gerät (11) über die Elektrode (15) des Instruments (14) durch das Plasma (19) und den Körper (18) des Patienten zu schließen, wobei das Plasma (19) zwischen der Elektrode (15) am distalen Ende des Instruments (14) und dem Gewebe (21) des Patienten gezündet ist.

5. Plasma (19) nach einem der vorstehenden Ansprüche, wobei das HF-Gerät (11) eingestellt ist, die Elektrode (15) mit einer Wechselspannung zu speisen,
die eine Hochfrequenz von mindestens 100 kHz, vorzugsweise zwischen einschließlich 200 kHz bis einschließlich 16 MHz, besonders bevorzugt zwischen 300 kHz und 500 kHz, beispielsweise 350 kHz, aufweist,
mit einer Mittelfrequenz (MF) gepulst ist, wobei die Mittelfrequenz zwischen einschließlich 5 kHz und einschließlich 100 kHz, besonders bevorzugt zwischen einschließlich 10 kHz und einschließlich 50 kHz, beispielsweise 20 kHz, beträgt,
wobei die Pulslänge (TPulsMF) der Mittelfrequenzpulse (40) eine oder mehrere HF-Perioden beträgt,
und mit einer Niederfrequenz (NF) gepulst ist, welche zwischen einschließlich 0,5 Hz und einschließlich 200 Hz, vorzugsweise zwischen einschließlich 20 Hz und einschließlich 150 Hz, insbesondere 100 Hz beträgt,
wobei die Pulslänge (TPulsNF) der Niederfrequenzpulse mindestens eine Mittelfrequenzperiode (MFP) beträgt, vorzugsweise zwischen einschließlich einer und einschließlich 50 Mittelfrequenzperioden (MFP), beispielsweise 20 Mittelfrequenzperioden (MFP).

6. Plasma (19) nach einem der vorstehenden Ansprüche, wobei das System (10) eine Vorrichtung (42) zur Regelung der Ausgangsleistung des HF-Geräts (11) aufweist.

7. Plasma (19) nach einem der vorstehenden Ansprüche, wobei die Gaszuführungseinrichtung (13) dazu eingerichtet ist, das Instrument (14) mit einem Fluss in einem Bereich von einschließlich einem Mindestgasfluss und einschließlich einem Gasfluss zu speisen, der drei Mal größer ist als der Mindestgasfluss.

8. Medium (21), insbesondere Flüssigkeit, beispielsweise Körperflüssigkeit, oder neoplastisches Gewebe, in welches mittels eines Plasmas (19) nach einem der vorstehenden Ansprüche Spezies (25), insbesondere Radikale, erzeugt und/oder eingebracht sind, zur Prävention von intraepithelialen Neoplasien, insbesondere durch antivirale Therapie, zur Behandlung von intraepithelialien Neoplasien aller Schweregrade, beispielsweise Carcinoma in situ Läsionen, und/oder zur Behandlung von invasiven epithelial erreichbaren Karzinomen.

9. Spezies (25), insbesondere Radikale, erzeugt mittels eines Plasmas (19) nach einem der Ansprüche 1 bis 7 zur Prävention von intraepithelialen Neoplasien, insbesondere durch antivirale Therapie, zur Behandlung von intraepithelialien Neoplasien aller Schweregrade, beispielsweise Carcinoma in situ Läsionen, und/oder zur Behandlung von invasiven epithelial erreichbaren Karzinomen.

10. System (10) zur Erzeugung eines Plasmas (19) nach einem der Ansprüche 1 bis 7 oder eines Mediums (21) nach Anspruch 8 oder von Spezies (25) nach Anspruch 9.

11. System (10) zur Erzeugung eines Plasmas (19), bevorzugt eines stromführenden Plasmas (19), bevorzugt eines Argon-Plasmas, mit einem medizinischen Instrument (14) mit einer Elektrode (15) und einem HF-Gerät (11) zur Bereitstellung einer Wechselspannung zur Speisung des Instruments (14) mit elektrischer Leistung, wobei das System (10) bevorzugt eine Gaszuführungseinrichtung (13) aufweist, welche eingestellt ist, um das Instrument (14) mit Gas, insbesondere Argon, zu speisen, wobei das System (10) eine Vorrichtung (42) zur Regelung der Ausgangsleistung, vorzugsweise der Ausgangswirkleistung, des HF-Geräts (11) auf einen Sollwert aufweist.

12. System (10) nach Anspruch 11, wobei das HF-Gerät (11) eingestellt ist, die Elektrode (15) mit einer Wechselspannung zu beaufschlagen,
die eine HF-Frequenz, beispielsweise von mindestens 100 kHz, vorzugsweise zwischen einschließlich 200 kHz bis einschließlich 16 MHz, besonders bevorzugt zwischen 300 kHz und 500 kHz, beispielsweise 350 kHz, aufweist,
und wobei die Wechselspannung mit einer Mittelfrequenz (MF) gepulst ist, wobei die Mittelfrequenz beispielsweise zwischen einschließlich 5 kHz und einschließlich 100 kHz, besonders bevorzugt zwischen einschließlich 10 kHz und einschließlich 50 kHz, beispielsweise 20 kHz beträgt, wobei die Pulslänge der Mittelfrequenzpulse vorzugsweise eine oder mehrere HF-Perioden beträgt,
und/oder wobei die Wechselspannung mit einer Niederfrequenz (NF) moduliert ist, welche beispielsweise zwischen einschließlich 0,5 Hz und einschließlich 200 Hz, vorzugsweise zwischen einschließlich 20 Hz und einschließlich 150 Hz, insbesondere 100 Hz beträgt, wobei die Pulslänge der Niederfrequenzpulse vorzugsweise mindestens eine Mittelfrequenzperiode beträgt, vorzugsweise zwischen einschließlich einer und einschließlich 50 Mittelfrequenzperioden, beispielsweise 20 Mittelfrequenzperioden,
wobei die Vorrichtung (42) dazu eingerichtet ist, die Ausgangsleistung, vorzugsweise die Ausgangswirkleistung, mittels der Anpassung der Modulation mit der Mittelfrequenz und/oder der Niederfrequenz zu regeln.

13. System (10) nach einem der Ansprüche 11 oder 12, wobei der Sollwert der Ausgangswirkleistung des HF-Geräts maximal 3,5 W, besonders bevorzugt maximal 2,5 W, ganz besonders bevorzugt maximal 2 W, noch weiter bevorzugt maximal 1 W beträgt.

14. Verfahren zur plasmabasierten Prävention von intraepithelialen Neoplasien, insbesondere durch antivirale Therapie, z.B. gegen das humane Papillomavirus, zur Behandlung von intraepithelialien Neoplasien aller Schweregrade, beispielsweise Carcinoma in situ Läsionen, und/oder zur Behandlung von invasiven epithelial erreichbaren Karzinomen, beispielsweise mittels des Plasmas (19) nach einem der Ansprüche 1 bis 7 und/oder mittels des Systems (10) nach einem der Ansprüche 11 bis 13, wobei das Verfahren das Überstreichen einer Gewebestelle (21) mit dem Plasma (19), welches eine Temperatur von größer oder gleich 45°C aufweist, mit einer Applikationsgeschwindigkeit gleich oder oberhalb eines Grenzwerts aufweist, so dass die Temperatur der Gewebestelle (21) unterhalb oder gleich einer Grenztemperatur bleibt.

15. Plasma zur Durchführung des Verfahrens gemäß Anspruch 14, beispielsweise Plasma nach Anspruch 1.
